# FASCICULE DE BREVET EUROPEEN

(11) **EP 2 459 229 B1**
(45) Date de publication et mention de la délivrance du brevet: **28.02.2018**
(21) Numéro de dépôt: 10745190.8
(22) Date de dépôt: 02.08.2010
(51) Int. Cl.: A61K 47/64

(54) **UTILISATION D'OLIGOMÈRES MIMES CONTRAINTS DE DIPEPTIDES ET TRIPEPTIDES EN TANT QU'AGENTS DE VECTORISATION**
VERWENDUNG VON MIMETISCHEN OLIGOMEREN MIT EINGESCHRÄNKTEN DIPEPTIDEN UND TRIPEPTIDEN ALS VEKTORISIERER
USE OF CONSTRAINED DIPEPTIDE AND TRIPEPTIDE MIMIC OLIGOMERS AS VECTORIZATION AGENTS

(30) Priorité: 31.07.2009 FR 0955427
(43) Date de publication de la demande: 06.06.2012
(73) Titulaire: Centre National de la Recherche Scientifique (C.N.R.S.), 75016 Paris (FR); Université de Montpellier I, 34000 Montpellier (FR); Institut National de la Santé et de la Recherche Médicale, 75013 Paris (FR)
(72) Inventeur: AMBLARD, Muriel, F-34730 Saint Vincent De Barbeyrargues (FR); MARTINEZ, Jean, F-34720 Caux (FR); VEZENKOV, Lubomir, F-34000 Montpellier (FR); HERNANDEZ, Jean-François, F-34150 Gignac (FR); GARCIA, Marcel, F-34730 Prades le Lez (FR); MAYNADIER, Marie, F-84400 Auribeau (FR)
(74) Mandataire: Lavoix
(86) Numéro de dépôt international: PCT/EP2010/061229
(87) Numéro de publication internationale: WO 2011/012729

(56) Documents cités:
- US-A1- 2003 191 049
- BALLET STEVEN ET AL: "Blood-brain barrier penetration by two dermorphin tetrapeptide analogues: role of lipophilicity vs structural flexibility." JOURNAL OF MEDICINAL CHEMISTRY 24 APR 2008, vol. 51, no. 8, 24 avril 2008 (2008-04-24), pages 2571-2574, XP002566595 ISSN: 0022-2623
- HEITZ FREDERIC ET AL: "Twenty years of cell-penetrating peptides: from molecular mechanisms to therapeutics." BRITISH JOURNAL OF PHARMACOLOGY MAY 2009, vol. 157, no. 2, 20 mars 2009 (2009-03-20), pages 195-206, XP8118202 ISSN: 1476-5381
- FARRERA-SINFREU JOSEP ET AL: "Cell-penetrating proline-rich peptidomimetics." METHODS IN MOLECULAR BIOLOGY (CLIFTON, N.J.) 2007, vol. 386, 2007, pages 241-267, XP8118206 ISSN: 1064-3745
- PUJALS ET AL: "Proline-rich, amphipathic cell-penetrating peptides" ADVANCED DRUG DELIVERY REVIEWS, ELSEVIER BV, AMSTERDAM, NL, vol. 60, no. 4-5, 17 novembre 2007 (2007-11-17), pages 473-484, XP022476839 ISSN: 0169-409X
- Josep Farrera-Sinfreu ET AL: "-Proline-Derived Peptides", Journal of the American Chemical Society, vol. 127, no. 26, 1 July 2005 (2005-07-01) , pages 9459-9468, XP055152904, ISSN: 0002-7863, DOI: 10.1021/ja051648k
- MAYNADIER MARIE ET AL: "Dipeptide mimic oligomer transporter mediates intracellular delivery of Cathepsin D inhibitors: A potential target for cancer therapy", JOURNAL OF CONTROLLED RELEASE, vol. 171, no. 2, 27 July 2013 (2013-07-27) , pages 251-257, XP028710780, ISSN: 0168-3659, DOI: 10.1016/J.JCONREL.2013.07.017

## Description

La présente invention concerne une nouvelle classe de composés capables de pénétrer dans les cellules biologiques et d'y véhiculer des molécules d'intérêts, tels que des médicaments ou des sondes biologiques.

Plus particulièrement, l'invention se rapporte à l'utilisation d'oligomères de motifs contraints de dipeptides et tripeptides en tant qu'agents de vectorisation.

Le problème du transport de substances actives au travers de la membrane plasmique et de leur accès aux divers compartiments intracellulaires est actuellement un problème majeur dans un grand nombre de thérapies (anticancéreuses, antivirale par exemple).
En effet, et même si la lipophilicité est un facteur de capture par la membrane, la molécule n'est pas garantie de traverser ladite membrane pour accéder au cytoplasme. Parmi les moyens actuellement utilisés pour introduire des substances dans les cellules, les peptides de translocation dénommés CPP pour « Cell-Penetrating-Peptides » (Advanced Drug Delivery Reviews, Volume 60, Numéros 4-5, Pages 447-614 (1 Mars 2008), Membrane Permeable Peptide Vectors: Chemistry and Functional Design for the Therapeutic Applications, Edité par S. Futaki) sont les vecteurs les plus utilisés. Depuis une dizaine d'années, ces vecteurs font l'objet de nombreux travaux pour l'intérêt qu'ils présentent dans la vectorisation d'anti-tumoraux, oligonucléotides antisens, acide peptido-nucléique (PNA), petits ARN interférents (ARNsi), peptides ou protéines.
Toutefois, la stabilité de ces composés de nature peptidique vis-à-vis des protéases, implique un risque de destruction rapide du conjugué vecteur/molécule active *in vivo.* Enfin, le caractère hydrophile des molécules de type poly-cationique, généralement utilisées dans ce domaine, ne permet pas la translocation de médicaments au travers de certaines barrières physiologiques comme la barrière hémato-encéphalique.

Toutes ces raisons rendent essentiel le développement de nouveaux vecteurs tant pour la recherche fondamentale (compréhension des mécanismes d'internalisation) que pour des utilisations thérapeutiques ou diagnostiques.
Ainsi, et bien que le domaine de vectorisation de molécules d'intérêt met déjà à disposition un certain nombre de composés, certains points doivent être résolus, parmi lesquels la biodisponibilité, la toxicité, un adressage spécifique des compartiments intracellulaires et la faisabilité au niveau industriel.

L'objet de la présente invention vise à résoudre certains de ces problèmes. Il implique l'utilisation d'un oligomère précédemment décrit (WO 01/51506) pour vectoriser des molécules d'intérêt. Ces oligomères sont connus pour être des mimes de polypeptides ou de protéines. Lesdits mimes de polypeptides ou de protéines sont plus stables que leurs analogues naturels dont ils diffèrent par la structure, notamment par la taille. En outre, un oligomère constitué seulement de quelques monomères, lié à une molécule d'intérêt, est capable de traverser une membrane plasmique.

### Résumé de l'invention :

La présente invention concerne l'utilisation d'un oligomère de formule (I') :

-X₁-(A)ₙ-X₂- (I')

pour préparer un principe actif (PA) vectorisé de formule (I) en liant (PA) à X₁ et/ou X₂ afin de faciliter l'entrée dudit principe actif dans des cellules biologiques, dans laquelle les unités récurrentes -(A)- et les termes X₁, A, X₂ et n sont tels que définis ci-dessous.

Les unités récurrentes -(A)-, indépendamment identiques ou différentes les unes des autres, représentent des mimes contraints de dipeptides ou tripeptides, avantageusement inducteurs de coude béta.

Le nombre d'unités récurrentes -(A)- de l'oligomère est défini par le nombre n; n est un nombre entier compris entre 2 et 40.

Dans la formule (I'), les unités récurrentes A, indépendamment identiques ou différentes les unes des autres, représentent des mimes contraints de dipeptides ou de tripeptides choisies parmi les formules (V) et (VI) suivantes :

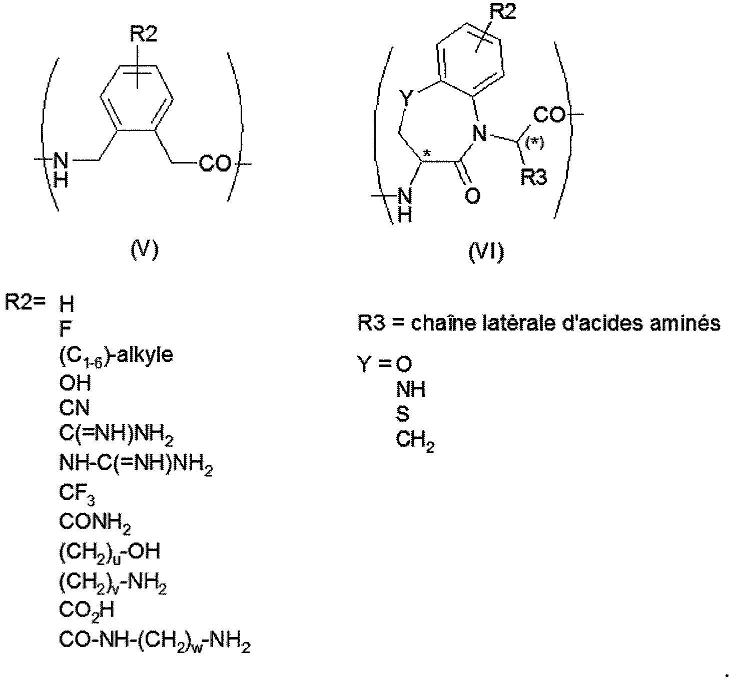

Dans la formule (I'), les termes X₁ et X₂, indépendamment l'un de l'autre, représentent un groupe espaceur ou une liaison.

Un autre objet de la présente invention concerne l'oligomère représenté par la formule générale (I):

R₆-X₁-(A)ₙ-X₂-R₇ (I)

dans laquelle les unités récurrentes -(A)-, et les termes n, A, X₁ et X₂ sont tels que définis pour la formule (I') et, l'un au moins de R₆ et R₇, est un principe actif (PA) ou un marqueur. Dans le cas où R₆ est un principe actif ou un marqueur, R₇ est choisi parmi les groupes hydroxy, C₁-C₆ alcoxy, aryl-(C₁-C₆ alcoxy)-, ou NH₂ ou R₇ représente un principe actif ou un marqueur identique ou différent de R₆. Dans le cas où R₇ est un principe actif ou un marqueur, R₆ est choisi parmi un atome d'hydrogène, un groupe C₁-C₆ alkyle, un groupe aryl-(C₁-C₆ alkyl)-, ou R₆ est un principe actif ou un marqueur identique ou différent de R₇.

Un autre objet de la présente invention concerne un procédé de synthèse pour obtenir l'oligomère de formule (I). Les techniques utilisées sont semblables à celles utilisées en synthèse peptidique.
Un autre objet de la présente invention concerne l'oligomère de formule (I) utilisé en tant que médicament.
Enfin la présente invention a également pour objet l'utilisation de l'oligomère de formule (I) pour faciliter l'entrée d'un principe actif (PA) ou d'un marqueur dans des cellules biologiques.

### Définitions

Les « mimes contraints » sont des fragments moléculaires permettant d'induire artificiellement des structures secondaires, telles que des hélices α, feuillet β ou coudes β, retrouvés dans des macromolécules telles que des protéines.

Un « dipeptide » est un fragment polymère de deux acides aminés reliés entre eux par une liaison amide, encore appelée liaison peptidique, issue d'une condensation entre l'amine d'un premier acide aminé et l'acide carboxylique d'un deuxième acide aminé. Un « tripeptide » est constitué par 3 acides aminés reliés entre eux par deux liaisons peptidiques.

Par groupement « C₁-C₆ alkyle », on entend une chaîne hydrocarbonée saturée, linéaire ou ramifiée, comportant de 1 à 6 atomes de carbone, comme par exemple un groupement méthyle, éthyle, isopropyle, *tertio*-butyle, pentyle, etc.

Par groupement « aryle », on entend un groupement aromatique, comportant de préférence de 5 à 10 atomes de carbone, comprenant un ou plusieurs cycles et comprenant éventuellement un ou plusieurs hétéroatome(s) en particulier un oxygène, un azote ou un soufre, comme par exemple un groupement phényle, furane, indole, pyridine, naphtalène, etc.

Le terme « chaîne latérale d'un acide aminé » représente le fragment porté par le carbone α d'un acide aminé. Par exemple, les chaînes latérales d'acides aminés naturels tels que la glycine, la valine, l'alanine et l'acide aspartique correspondent à l'atome d'hydrogène, aux groupes isopropyle, méthyle et CH₂COOH respectivement.

Le terme acide aminé naturel représente entre autres les acides aminés suivants : la glycine (Gly), l'alanine (Ala), la valine (Val), la leucine (Leu), l'isoleucine (Ile), la sérine (Ser), la thréonine (Thr), la phénylalanine (Phe), la tyrosine (Tyr), le tryptophane (Trp), la cystéine (Cys), la méthionine (Met), la proline (Pro), l'hydroxyproline, l'acide aspartique (Asp), l'asparagine (Asn), la glutamine (Gln), l'acide glutamique (Glu), l'histidine (His), l'arginine (Arg), l'ornithine, et la lysine (Lys).

Les chaînes latérales d'autres acides aminés peuvent être inclus dans la définition, tels que celles des acides aminés suivants: acide 4-amino tétrahydropyran-4-carboxylique, allylglycine, acide diamino butyrique, acide diamino propionique, aminosérine, acide aminobutyrique, amino butylglycine, phénylglycine, 4-chloro-phénylalanine, 4-nitro-phénylalanine, citrulline, cyclohexylalanine, thiénylalanine, et de leurs semblables.

Les chaînes latérales des acides aminés peuvent être protégées par des groupements protecteurs (P) et plus particulièrement N-protecteurs, O-protecteurs ou S-protecteurs lorsque ces chaînes contiennent les hétéroatomes correspondants.

Les groupements protecteurs (P) sont des groupements connus de l'homme du métier. Ces groupements protecteurs et leur utilisation sont décrits dans des ouvrages tels que par exemple Greene, "Protective Groups in Organic Synthesis", Wiley, New York, 2007 4ème édition ; Harrison et al. "Compendium of Synthetic Organic Methods", Vol. 1 à 8 (J. Wiley & sons, 1971 to 1996); Paul Lloyd-Williams, Fernando Albericio, Ernest Giralt, "Chemical Approaches to the Synthesis of Peptides and Proteins", CRC Press, 1997 ou Houben-Weyl, "Methods of Organic Chemistry, Synthesis of Peptides and Peptidomimetics", Vol E 22a, Vol E 22b, Vol E 22c, Vol E 22d., M. Goodmann Ed., Georg Thieme Verlag, 2002. Selon que ces groupements protecteurs sont portés par un atome d'azote, ils seront désignés en tant que groupements N-protecteurs. Il en est de même pour les groupements S-protecteurs, O-protecteurs, etc. Par exemple, un hydroxy peut être protégé par un groupement trityl, ou un acide carboxylique peut être protégé sous forme d'un ester tert-butylique. Si on fait une synthèse sur support solide, c'est la résine qui sert de groupement protecteur à la fonction carboxylique C-terminale. La protection du groupe amino de l'acide aminé peut être effectuée par exemple par un groupe tert-butyloxycarbonyle (désigné ci-après par Boc-) ou un groupe -9-fluorénylméthyloxycarbonyle (désigné ci-après par Fmoc) représenté par la formule :

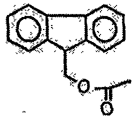

La protection est effectuée selon les procédés connus de l'art antérieur. Par exemple, la protection par le groupe Boc- peut être obtenue en faisant réagir l'acide aminé avec le di-tert-butylpyrocarbonate (Boc₂O).

Le terme « cycle hydrocarboné monocyclique ou polycyclique saturé » représente indifféremment dans la présente invention des groupes hydrocarbonés saturés comprenant un ou plusieurs cycles, avantageusement 1, 2 ou 3 cycles, chacun des cycles comprenant de 3 à 10 atomes de carbones inscrit dans ledit cycle.
Le terme « cycle hydrocarboné monocyclique ou polycyclique insaturé » représente indifféremment dans la présente invention des groupes hydrocarbonés insaturés comprenant un ou plusieurs cycles, avantageusement 1, 2 ou 3 cycles, avec au moins un des cycles comprenant au moins une insaturation, chacun des cycles comprenant de 3 à 10 atomes de carbones inscrits dans ledit cycle. En outre, le terme « cycle hydrocarboné monocyclique ou polycyclique insaturé » comprend les groupements cycliques aromatiques, c'est-à-dire les aryles, définis plus haut.
Dans le cas où le groupe polycyclique comprend 2 cycles, lesdits cycles peuvent être fusionnés, pontés, liés entre eux par une jonction spiro, ou l'un des atomes de carbone d'un des cycles forme une liaison covalente avec un atome de carbone de l'autre cycle.

Par exemple, un groupe hydrocarboné bicyclique dans lequel chaque cycle est un cycle constitué de 6 atomes de carbone peut être condensé des façons suivantes: 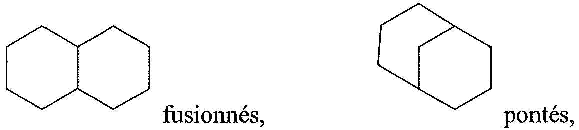 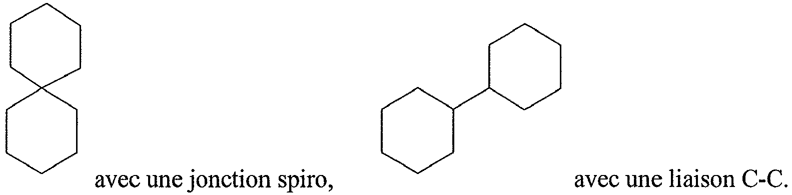 Dans le cas où le groupe polycyclique comprend plus de 2 cycles, l'homme du métier comprendra qu'il peut exister une combinaison de ces configurations de cycles.
Il doit être compris aussi qu'un ou plusieurs de ces cycles peuvent être hétérocycliques, ce qui veut dire que les cycles incorporent un ou plusieurs hétéroatomes, choisis avantageusement parmi les atomes d'azote, d'oxygène, de soufre.
Des exemples de cycles hydrocarbonés ou hétérocycliques, mono ou polycycliques, saturés ou insaturés, comprennent les groupes cyclopentyle, le cyclohexyle, le cycloheptyle, adamantyle, pipéridinyle ou norbornyle.

Un « groupe espaceur » selon l'invention est un fragment moléculaire permettant de relier l'oligomère -A- à un principe actif (PA). Avantageusement le groupe espaceur permet de diminuer la gêne stérique entre l'oligomère et le principe actif. Plus avantageusement, le groupe espaceur permet de relarguer le principe actif dans le ou les organites cibles de la cellule biologique - par exemple dans le lysosome, la mitochondrie ou l'appareil de Golgi. Le groupe espaceur est constitué d'au moins deux atomes pouvant être identiques ou différents.
Avantageusement, le groupe espaceur est un enchaînement de groupes limité en taille entre 1-50 atomes au total, qui permet d'éloigner le principe actif ou le marqueur de l'oligomère.

Avantageusement, le groupe espaceur est une chaîne hydrocarbonée comprenant un ou plusieurs groupements choisis parmi : C₁-C₆ alkyle, aryle, aryl-(C₁-C₆alkyle), cycle hydrocarboné monocyclique ou polycyclique saturé, cycle hydrocarboné monocyclique ou polycyclique insaturé, C₁-C₆ alcoxy, aryl-(C₁-C₆ alcoxy), acides aminés, éventuellement substitués par un ou des atomes d'hydrogène ou par un ou des C₁-C₄-alkyles.
Avantageusement, le groupe espaceur est l'éthylène glycol ou un polyéthylène glycol, ou un peptide ou un acide aminé qui peuvent éventuellement être substitués. Avantageusement, dans le cas où le groupe espaceur est un polyéthylène glycol, ledit polyéthylène glycol comprend de 2 à 10 unités éthylène glycol. Avantageusement, dans le cas où le groupe espaceur est un peptide, celui-ci comprends de 2 à 15 acides aminés, plus avantageusement de 2 à 10 acides aminés et encore plus avantageusement de 2 à 8 acides aminés.
Avantageusement dans le cas où le groupe espaceur est un acide aminé, ledit groupe espaceur est un acide aminé dont les fonctions acide carboxylique et amine sont séparées par une chaîne hydrocarbonée comprise entre 2 et 10 atomes de carbones, un substrat de protéase, un pont disulfure. Ces deux derniers espaceurs permettent de relarguer le principe actif, le premier par clivage par une protéase cible et le second par réduction (système glutathion). D'autres techniques de relarguage de principes actifs, connues de l'homme du métier, peuvent être appliquées à la présente invention.
Avantageusement le groupe espaceur comprend ou consiste en un groupement hydrophile. De tels groupements hydrophiles peuvent inclure des acides aminés de la série L ou de la série D, naturels ou non naturels, et permettent une meilleure solubilité du principe actif vectorisé. Par exemple des acides aminés connus pour aider la solubilisation, tels que l'arginine et/ou la lysine, peuvent être insérés. Avantageusement, ces acides aminés sont de la série D afin que ces groupements additionnels ne soient pas facilement hydrolysés in vivo.
Dans un mode de réalisation particulier de l'invention, le groupement espaceur comprend au moins un acide aminé.

Dans un mode de réalisation particulier de l'invention, le groupement espaceur consiste en des acides aminés.

Dans un mode de réalisation particulier de l'invention, le groupe espaceur comprend « -D-Arg-O₂Oc- » dans lequel D-Arg représente la D-Arginine, O₂Oc représente le 8 amino-3,6-dioxaoctanoyle.

Dans un mode de réalisation particulier de l'invention, le groupe espaceur consiste en « -D-Arg-O₂Oc- » dans lequel D-Arg représente la D-Arginine, O₂O_{C} représente le 8 amino-3,6-dioxaoctanoyle.

Dans un mode de réalisation particulier de l'invention, le groupe espaceur comprend « -D-Lys-O₂Oc- » dans lequel D-Lys représente la D-Lysine, O₂Oc représente le 8 amino-3,6-dioxaoctanoyle.

Dans un mode de réalisation particulier de l'invention, le groupe espaceur consiste en « -D-Lys-O₂Oc- » dans lequel D-Lys représente la D-Lysine, O₂Oc représente le 8 amino-3,6-dioxaoctanoyle.

Les techniques d'aide à la solubilisation/solubilité de principes actifs, sont bien connues de l'homme du métier et applicables aux composés de formule (I) en agissant au niveau des groupes espaceurs.

Par groupement « C₁-C₆ alcoxy », on entend, au sens de la présente invention, un groupe C₁-C₆ alkyle, tel que défini ci-dessus, lié à la molécule par l'intermédiaire d'un atome d'oxygène. A titre d'exemple, on peut citer les groupes méthoxy, éthoxy, propoxy, isopropoxy, butoxy ou encore tert-butoxy. « tBu » désigne tert-butyle.

Par groupement « aryl-(C₁-C₆ alcoxy) », on entend, au sens de la présente invention, un groupe aryle, tel que défini ci-dessus, lié à la molécule par l'intermédiaire d'un groupement C₁-C₆ alcoxy. A titre d'exemple, on peut citer les groupes benzyloxy, phényléthoxy, ou encore phénylpropoxy.
Par groupement « aryl-(C₁-C₆alkyle) », on entend, au sens de la présente invention, un groupe aryle, tel que défini ci-dessus, lié à la molécule par l'intermédiaire d'un groupement C₁-C₆ alkyl. A titre d'exemple, on peut citer les groupes benzyl, phényl-éthyle, phényl-propyle.

### Description détaillée

Les unités récurrentes -A- dans les formules (I) et (I') peuvent comprendre un ou plusieurs centres asymétriques, qui peuvent être de configuration R ou de configuration S.

Les unités récurrentes A, indépendamment identiques ou différentes les unes des autres, représentent des mimes contraints de dipeptides ou de tripeptides choisies parmi les formules (V) et (VI) telles que définies ci-dessus ou ci-dessous.

Comme exemples de groupements -A- qui présentent des propriétés de mimes contraints de dipeptides ou de tripeptides et qui peuvent être inducteurs de coude β, on peut citer les groupes suivants : 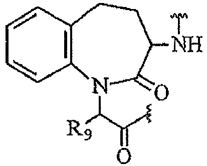 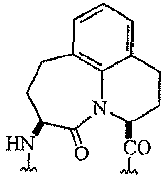 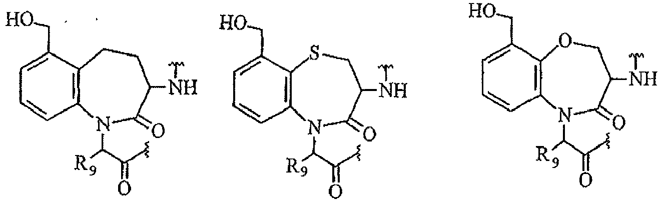 dans lesquels :
- R2 représente H, C₁-C₆ alkyle, nitrile, -NH-C(=NH)NH₂, -C(=NH)NH₂, -(CH₂)ᵤOH, -CO₂H, -CONH₂, F, CF₃, -(CH₂)ᵥNH₂, et/ou-CONH(CH₂)_{w}NH₂, u, v et w étant des nombres entiers compris entre 0 et 10.
- R3, et les cas échéants R₉ et R₁₀, sont choisis indépendamment les uns des autres parmi les groupes constituants les chaînes latérales des acides aminés, par exemple H, CH₃-, (CH₃)₂CH-, CH₃-(CH₂)₃- ou C₆H₅-CH₂-;
- m et les substituants X, Y et Z pour chaque composé qui les contient, sont définis de manière spécifique, et

Dans les composés ci-dessus comprenant des centres asymétriques, lesdits centres asymétriques peuvent être de configuration R ou S.

Dans les formules (I) ou (I'), si n est trop grand, alors il peut subvenir des problèmes de synthèse et/ou de solubilité. Si n est trop petit, l'oligomère ne permet pas le passage au travers d'une membrane biologique cellulaire. Avantageusement, pour les formules (I) et (I'), n est compris entre 2 et 10 et encore plus avantageusement, n est compris entre 4 et 8

Avantageusement, les oligomères de formules (I) et (I') peuvent être des hétérooligomères, c'est-à-dire comprenant au moins deux types différents d'unités récurrentes. Avantageusement, l'oligomère (I) ou (I') de la présente invention est un hétérooligomère où -(A)ₙ- présente la formule (VII)

-[B-D]ₙ- (VII)

dans laquelle:
- les unités récurrentes B et D sont différentes l'une de l'autre, etb représentent des mimes contraints de dipeptides ou de tripeptides choisies parmi les formules (V) et (VI).

Avantageusement, les deux unités -(B)- et -(D)- sont des unités de formule (VI) différentes l'une de l'autre.
Avantageusement, l'un desdits dérivés de formule (VI) est un composé de formule (VIII) :

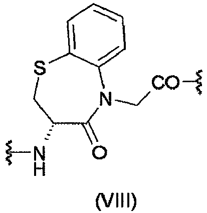

Avantageusement, dans les oligomères (I) et (I'), les groupes espaceurs X₁ et X₂, indépendamment l'un de l'autre sont une liaison ou choisis parmi les groupes suivants :

-NH-(CH₂CH₂O)₂CH₂CO- (IX)

-NH-(CH₂)₅CO- (X)

Le principe actif (PA) est une molécule connue pour son action biologique, en particulier utilisé en thérapie médicale humaine. Le principe actif peut aussi être utilisé pour le diagnostic de maladies. Le principe actif est la pepstatine ou la [Val¹]-pepstatine. D'une façon générale, l'objet de la présente invention permet de faciliter le passage de la membrane cellulaire d'un principe actif nécessitant un tel transport. Ladite membrane cellulaire peut être au niveau des tissus ciblés par le principe actif quelque soit le mode d'administration, et/ou au niveau du tractus digestif (dans le cas d'une administration par voie orale), et/ou au niveau d'un pathogène (bactérie, parasite, champignon).
Les principes actifs de l'oligomère de formule (I) sont des médicaments pour le traitement de maladies lysosomales, du cancer, ou de la maladie d'Alzheimer.
Les oligomères de formule (I) peuvent ainsi être utiles en tant que médicament, notamment dans le traitement d'un cancer dans lequel la cathepsine D est surexprimée.
R₆ et/ou R₇ peuvent être un ou des marqueurs afin de tracer l'oligomère.
Ces marqueurs peuvent être des marqueurs biotinylés, des marqueurs colorés, des marqueurs fluorescents, des marqueurs à métaux complexés tel que les complexes d'argent, des marqueurs pour l'électrophorèse en particulier 2D, des marqueurs d'ARN ou d'ARNt c'est-à-dire se complexant à de l'ARN ou de l'ARNt, des marqueurs détectables en UV-VIS, des marqueurs avec du xénon encapsulé ou pour encapsuler du xénon, des marqueurs pour toute technique d'imagerie médicale, et leurs semblable.
Dans la présente invention, le marqueur est ainsi choisi parmi les composés suivants : fluorescéine, sel sodique de fluorescéine, 4',5'-Bis[*N,N-*bis(carboxyméthyl)-aminométhyl]fluorescéine, acide 6-[fluorescéine-5(6)-carboxamido]héxanoique, acide 6-[fluorescéine-5(6)-carboxamido]héxanoique ester *N*-hydroxysuccinimide de fluorescéin-5(6)-isothiocyanate, fluorescéin-α-D-N-acetylneuraminide-polyacryl-amide, amidite de fluorescéine, fluorescéine-di(β-D-galactopyranoside), fluorescéin-di-(β-D-glucopyranoside), diacétate de fluorescéine, diacétate fluorescéin-5(6)-isothiocyanate, diacétate de fluorescéin-5-maleimide, diacétate de fluorescéin-6-isothiocyanate, dibutyrate de fluorescéine, fluorescéine dilaurate, sel de diphosphate de fluorescéine triammonium, acide fluorescéin-hyaluronic, isothiocyanate de fluorescéine- Dextran 500000-Conjugué, isomer I de la isothiocyanate de fluorescéine, isothiocyanate de fluorescéine-dextran, acétate de mercure-fluorescéine, chlorhydrate de mono-*p-*guanidinobenzoate-fluorescéine, *O*,*O'*-diacrylate de fluorescéine, fluorescéine *O,O'*-dimethacrylate, fluorescéine o-acrylate, fluorescéine *O*-méthacrylate, ester N-hydroxysuccinimide de fluorescéine, fluorescéine-5-thiosemicarbazide, fluorescéin-α-D-galactosamine polyacrylamide, fluorescéin-α-D-mannopyranoside-polyacrylamide, 4(5)-(iodoacétamido)-fluorescéine, 5-(Bromométhyl)fluorescéine, 5-(Iodoacétamido)fluorescéine, diacétate de l'ester *N-*succinimidyl-5-Carboxy-fluorescéine, diacétate de l'ester *N*-succinimidyl-6-Carboxy- fluorescéine, Aminophényl-fluorescéine, Biotin-4- fluorescéine, hydroxyphényl-fluorescéine, MTS-4- fluorescéine, chlorhydrate de poly(fluorescéine-isothiocyanate allylamine), poly(fluorescéine-*O*-acrylate), poly(fluorescéine-*O*-méthacrylate), acétate de PPHT-fluorescéine, chlorhydrate de 5-([4,6-dichlorotriazin-2-yl]amino) fluorescéine, chlorhydrate de 6-([4,6-dichlorotriazin-2-yl]amino) fluorescéine, poly[(méthylméthacrylate)-*co*-( fluorescéine-*O*-methacrylate)], poly[méthylméthacrylate-*co*-(fluorescéine *O-*acrylate)], 5(6)-(Biotinamidohexanoylamido)pentylthioureidylfluorescéine, N-(5-fluorescéinyl)maleimide, sel disodique de Mercure-dibromo-fluorescéine, fluorescéin-di-[méthylène-N-méthylglycine], sel disodique de 2',4',5',7'-tétrakis-(acétoxymercuro)-fluoroscéine, l'érythrosin B, l'éthyl eosin, 5-carboxy fluorescéine, ester *N*-succinimidyl de 5-carboxy fluorescéine ester perchlorate de, rhodamine B octadecyl, ester N-hydroxysuccinimide de 6-Carboxy-fluorescéine, dibenzyl fluorescéine, rhodol, 6-amino fluorescéine, rhodamine 6G, la rhodamine B ou encore la rhodamine 123.
La présente invention décrit par ailleurs que le ou les principes actifs peuvent être substitués par un ou plusieurs groupements hydrophiles. Ces groupements hydrophiles peuvent par exemple permettre une meilleure solubilité du principe actif vectorisé, voire du principe actif seul. Ces groupements hydrophiles comprennent au moins un acide aminé, de préférence consiste en des acides aminés.
Avantageusement, ces groupements hydrophiles comprennent au moins un acide aminé acylé, préférentiellement acétylé.
Avantageusement, ces groupements hydrophiles comprennent des acides aminés et présentent une fonction amine terminale acylée, préférentiellement acétylée. Avantageusement, ces groupements hydrophiles consistent en des acides aminés et présentant une fonction amine terminale acylée, préférentiellement acétylée. Avantageusement, ces groupements hydrophiles comprennent un acide aminé et présentant une fonction amine terminale acylée, préférentiellement acétylée. Avantageusement, ces groupements hydrophiles comprennent « Ac-D-Arg-O₂Oc- » dans lequel Ac représente un groupement acétyle, D-Arg représente la D-Arginine, 020c représente le 8 amino-3,6-dioxaoctanoyle.
Avantageusement, ces groupements hydrophiles consistent en « Ac-D-Arg-O₂Oc- » dans lequel Ac représente un groupement acétyle, D-Arg représente la D-Arginine, O2Oc représente le 8 amino-3,6-dioxaoctanoyle.
Avantageusement, ces groupements hydrophiles comprennent « Ac-D-Lys-O₂Oc- » dans lequel Ac représente un groupement acétyle, D-Lys représente la D-Lysine, 020c représente le 8 amino-3,6-dioxaoctanoyle.

Avantageusement, ces groupements hydrophiles consistent en « Ac-D-Lys-O₂Oc- » dans lequel Ac représente un groupement acétyle, D-Lys représente la D-Lysine, O₂Oc représente le 8 amino-3,6-dioxaoctanoyle.
De tels groupements hydrophiles peuvent inclure des acides aminés de la série L ou de la série D, naturels ou non naturels. Avantageusement, des acides aminés connus pour aider la solubilisation, tels que l'arginine et/ou la lysine peuvent être ajoutés. Avantageusement, ces acides aminés sont de la série D et l'amine terminale du groupement hydrophile est substituée par un groupement acyle, afin que ces groupements hydrophiles ne soient pas facilement hydrolysés in vivo.
Les techniques d'aide à la solubilisation/solubilité de principes actifs, sont bien connues de l'homme du métier.

La présente invention a également pour objet un procédé de synthèse pour obtenir les oligomères de formules (I).
Les techniques utilisées sont semblables à celles utilisées en synthèse peptidique. Une, plusieurs ou toutes les étapes peuvent être effectuées en phase liquide.
Une, plusieurs ou toutes les étapes peuvent être effectuée sur support solide.
Un oligomère (I) ou (I') est obtenu par polymérisation d'au moins un acide aminé mime contraint de dipeptide ou tripeptide, pouvant être un inducteur de coude β, et qui répond à la formule :

HA-OH (XI)

dans laquelle la groupe A a la même signification que dans l'oligomère (I) ou (I').
Ces monomères peuvent être synthétisés par des procédés décrits dans l'art antérieur. Par exemple, Amblard, et al., J. Med. Chem. , 1999, 42, 4193-4201, décrivent la synthèse de la (3S) [amino]-5-(carbonylméthyl)-2,3-dihydro-1,5-benzo-thiazépin-4 (5H) -one et d'autres monomères.

Avantageusement la synthèse de l'oligomère de formule (I) se fait en deux grandes étapes (α) et (β) : d'abord (α) par une polymérisation d'unités récurrentes avec ou non des groupements X₁ et/ou X₂ liés aux unités récurrentes N-terminale et/ou C-terminale, puis dans une deuxième étape (β) par liaison du principe actif sur l'oligomère obtenu. Dans les deux étapes, une sélection des fonctions chimiques réactives, telles certains hydroxyles, acides carboxyliques, amines, etc, sont protégées par des groupements protecteurs ou des techniques connues de l'homme du métier, qui peuvent être retrouvées dans : Greene, "Protective Groups in Organic Synthesis", Wiley, New York, 2007 4ème édition; Harrison et al. "Compendium of Synthetic Organic Methods", Vol. 1 à 8 (J. Wiley & sons, 1971 à 1996); Paul Lloyd-Williams, Fernando Albericio, Ernest Giralt, "Chemical Approaches to the Synthesis of Peptides and Proteins", CRC Press, 1997 ou Houben-Weyl, "Methods of Organic Chemistry, Synthesis of Peptides and Peptidomimetics", Vol E 22a, Vol E 22b, Vol E 22c, Vol E 22d., M. Goodmann Ed., Georg Thieme Verlag, 2002.

Pour la première étape (α), de manière identique à la synthèse peptidique classique, un sens de synthèse peut être donné. Dans les structures données ci-dessus, une liaison amide peut être formée entre deux unités récurrentes pour former un oligomère, par l'utilisation d'un agent de couplage. Les unités récurrentes ayant chacune une extrémité COOH et une extrémité NH₂ protégées ou non, avant réaction, la partie restante non réactive (donc protégée) est référée comme extrémité C-terminale ou N-terminale respectivement. Ainsi un sens de synthèse peut être défini selon l'extrémité par lequel l'homme du métier souhaite agrandir l'oligomère : synthèses C→N ou N→C.

Exemple de structure pouvant être produite par le présent procédé : 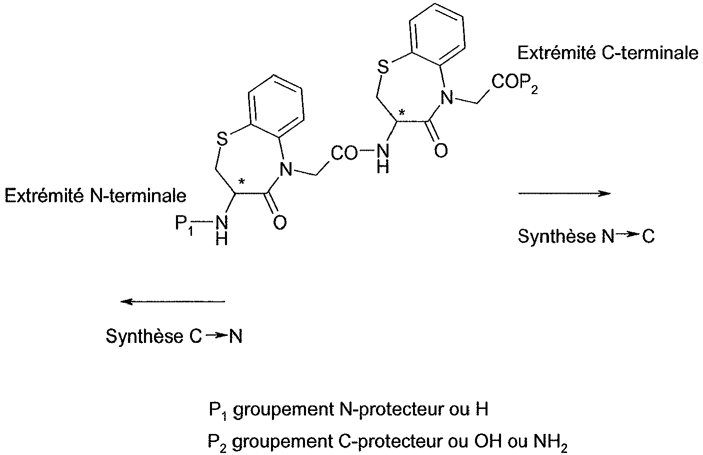 Ensuite, un des groupements protecteurs P₁ ou P₂ est clivé par des techniques connues de l'homme du métier et une seconde réaction de couplage peut être effectuée. La polymérisation contrôlée des unités récurrentes est ainsi obtenue par des étapes successives de couplage-déprotection permettant d'obtenir un oligomère de taille voulue et de façon univoque. L'oligomère final peut être déprotégé *in fine* soit sélectivement sur la fonction carboxylique, soit sur la fonction aminée, soit totalement.
Ainsi dans le cas présent, dans l'étape (α), que ce soit en phase liquide ou en phase solide, la synthèse peut se faire dans le sens C→N ou N→C. Le sens de la synthèse peut être dicté par la molécule finale voulue ou par des facteurs de synthèse autres, tels que des risques d'épimérisation. Quelque soit le sens de la synthèse l'homme de l'art connaît les stratégies (il y en a plusieurs) pour incorporer le principe actif (PA) aussi bien au niveau N-terminal sur X₁ qu'au niveau C-terminal sur X₂. La molécule finale est totalement ou partiellement déprotégée selon les techniques connues de l'homme du métier.
Avantageusement, un oligomère (I) ou (I') est obtenu par un procédé de polymérisation en phase solide, selon une stratégie classique de synthèse peptidique sur support: une résine portant une fonction aminée est condensée à un acide aminé protégé sur sa fonction amine, puis le fragment ainsi fixé sur la résine est allongé depuis le côté C-terminal vers le côté N-terminal, avant d'être séparé de la résine.
Dans le procédé de polymérisation en phase solide, chaque étape de couplage d'un composé (XI) préalablement protégé sur sa fonction aminée (XI') comprend la réaction de couplage proprement dite en présence d'un agent de couplage, le lavage du produit obtenu après le couplage, puis la déprotection du groupe amino de l'unité -A- fixée.
Dans la présente invention, le procédé de préparation de l'oligomère de formule (I) se fait par les étapes successives suivantes:
a) polymérisation par une stratégie de synthèse peptidique sur support solide, comprenant la réaction de l'unité de mime contraint de dipeptides ou de tripeptides, avantageusement inducteur de coude béta, de formule (XI') suivante:

   P-A-OH (XI')

   dans lequel le groupement P est un groupement N-protecteur, l'unité récurrente -A- et les termes A, sont tels que définis ci-dessus pour la formule (I') et convenablement protégés par des groupements protecteurs, pour synthétiser le produit (XII) suivant:

   H-(A)ₙ-X₂-R₈-(SS) (XII)

   dans lequel,
   - n et X₂ sont tels que définis ci-dessus;
   - R₈ est un groupe précurseur de R₇ avant clivage, R₇ est tel que défini ci-dessus;
   - (S S) est le support solide ;
a2) éventuellement, si X₁ n'est pas une liaison, réaction de couplage entre P-X₁-OH, dans lequel le groupement P est un groupement N-protecteur et X₁ est tel que défini ci-dessus, et l'amine N-terminale du produit (XII), suivie d'une étape de déprotection de l'extrémité N-terminale pour synthétiser le produit (XII') suivant :

   H-X₁-(A)ₙ-X₂-R₈-(SS) (XII')
b) réaction de couplage entre un principe actif (PA) et l'amine N-terminale du produit (XII'), pour synthétiser le produit (XIII) suivant :

   (PA)-X₁-(NR₂-R-A-R'-CO)ₙ-X₂-R₈-(SS) (XIII)

   dans lequel,
   (PA) est tel que défini ci-dessus,
c) une réaction de clivage permettant de libérer l'oligomère de formule (I) du support solide à partir du produit de formule (XIII).

Dans le cadre de la présente invention, les termes supports solides, résines supports ou résines sont équivalents.
Avantageusement, le procédé comprend une étape (a1) antérieure à l'étape (a) de couplage d'un groupe espaceur X₂ sur le support solide

Pour l'étape (a), on peut utiliser comme résine support toute résine utilisée de manière classique dans les synthèses peptidiques. A titre d'exemple, on peut citer une résine 4-méthylbenzhydrylamine (désignée ci-après par résine MBHA), ou une résine dite Merrifield, qui est un copolymère de styrène et de divinylbenzène fonctionnalisée par le chlorobenzyle. Les deux résines sont des résines commerciales distribuées notamment par les sociétés Novabiochem et Bachem. On peut également citer les résines PAL-PEG-PS, qui sont des copolymères d'acide 5-(4-aminométhyl-3,5-diméthoxyphénoxy)valérique - polyéthylène glycol - polystyrène.
Des résines de type «Rink amide» (4-[2',4'-diméthoxyphényl-(9-fluorénylméthyloxycarbonyl)aminométhyl]phénoxy-aminométhyl polystyrène) et « Wang » (alcool 4-benzyloxy-benzyl polystyrène) peuvent également être utilisées comme support solide (SS).
Avantageusement les résines utilisées sont de type « Wang » ou « Rink Amide ».

L'agent de couplage peut être choisi parmi les agents de couplage classiquement utilisés en synthèse peptidique. On peut citer par exemple : N,N'-dicyclohexylcarbodiimide (DCC), 1-éthyl-3-(3'-diméthylaminopropyl)-carbodiimide hydrochlorure (EDC), O-(1H-benzotriazol-1-yl)-N,N,N',N'-tétraméthyluronium tétrafluoroborate (TBTU), benzotriazol-1-yl-oxytris(diméthylamino)phosphonium hexafluorophosphate (BOP), O-(7-azabenzotriazol-1-yl)-1,2,3-tétraméthyluronium hexafluorophosphate (HATU), O-benzotriazol-1-yl-N,N,N',N'-tétramethyluronium hexafluorophosphate (HBTU), N-hydroxy- 5-norbornène-2,3-dicarbodiimide, ou tout autre agent de couplage dans un solvant tel que l'éther, l'acétone, le chloroforme, dichlorométhane, acétate d'éthyle, diméthylformamide (DMF), tétrahydrofurane (THF), acétonitrile, diméthylsulfoxyde (DMSO), N-méthyl pyrrolidone (NMP), refroidi ou à température ambiante, préférentiellement en présence d'un catalyseur d'acylation tel que le N-hydroxybenzotriazole (HOBt), 1-hydroxy-7-azabenzotriazole (HOAt), N-hydroxysuccinimide et leurs semblables.
On peut également utiliser pour le couplage, les anhydrides symétriques préformés à partir d'un monomère (XI') et du DCC.
Le lavage des produits obtenus après chaque phase de couplage peut être effectué à l'aide des solvants utilisés classiquement dans les synthèses peptidiques en phase solide.
A titre d'exemple, on peut citer le diméthylformamide (DMF), le méthanol et le dichlorométhane (DCM).
Le réactif utilisé pour la déprotection du groupe aminé après une étape de couplage dépend de l'agent de protection utilisé. Par exemple, si la protection est effectuée par un groupe Boc, la déprotection est effectuée avantageusement à l'aide d'une solution d'acide trifluoroacétique (TFA). Si la protection est effectuée par un groupe Fmoc, la déprotection peut être effectuée par la pipéridine. De manière générale, les groupes protecteurs et les réactifs de déprotection utilisés de manière connue lors des synthèses peptidiques en phase solide peuvent être utilisés lors de la synthèse des oligomères de la présente invention.

L'oligomère peut avantageusement être séparé de la résine par traitement par un acide. A titre d'exemple, on peut citer l'acide trifluoroacétique en présence de tris-isopropylsilane et d'eau ou l'acide fluorhydrique en présence d'anisole, suivant le type de résine utilisée. Dans ces conditions, la protection du groupe amino terminal avant séparation de la résine peut être effectuée par un groupe uréthane stable dans les conditions acide de clivage ou par un groupe acyle stable dans les mêmes conditions. Lorsque la séparation est effectuée par l'acide trifluoroacétique, le groupe amino est protégé par exemple par un groupe Fmoc ou Boc définis précédemment.
La préparation des oligomères en phase solide est réalisée de manière avantageuse dans un synthétiseur automatique utilisé de manière classique pour la synthèse des peptides sur support solide. Dans ce type d'appareil, la succession des différentes opérations de couplage, de lavage, de déprotection, est pilotée par un ordinateur.

### Légendes des figures :

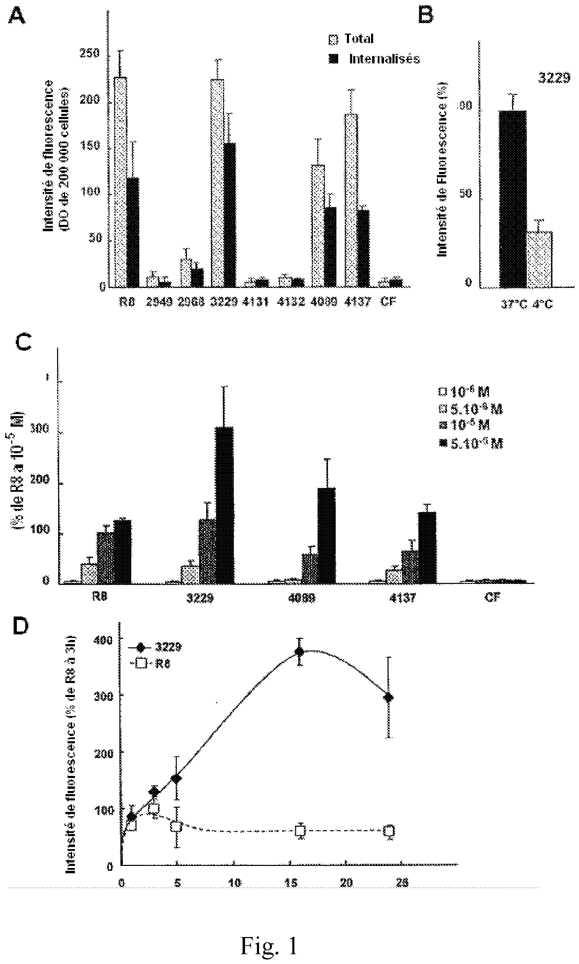

La Figure 1 décrit l'internalisation des oligomères dans les cellules cancéreuses mammaires humaines de la lignée MDA-MB-231. La Figure 1A décrit l'intensité de la fluorescence émise par les oligomères marqués par de la fluorescéine. La Figure 1B décrit l'intensité de la fluorescence de JMV 3229 en fonction de la température. La Figure 1C décrit l'intensité de la fluorescence en fonction de la concentration en oligomères. La Figure 1D décrit l'évolution de la fluorescence en fonction du temps (cinétique).

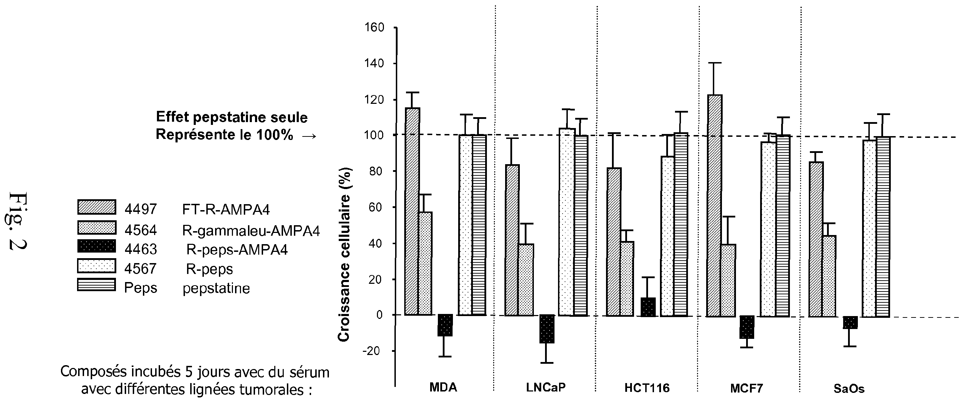

La Figure 2 décrit la croissance cellulaire (en pourcentage de nombre de cellules par rapport à un témoin pepstatine) de cellules cancéreuses (mammaires MDA-MB-231 et MCF7, prostatiques LNCaP, coliques HCT116, ostéoblastiques SaOs2) en fonction de leur incubation pendant 5 jours avec 1 oligomère d'AMPA (4497), de la pepstatine seule (Peps), de la [Val¹]-Pepstatine reliée à de l'arginine (4567) et deux conjugués inhibiteurs de cathepsine D-oligomère d'AMPA reliés à un résidu arginine (4564 et 4463). Les pourcentages négatifs correspondent à une diminution du nombre de cellules par rapport à l'ensemencement cellulaire initial.

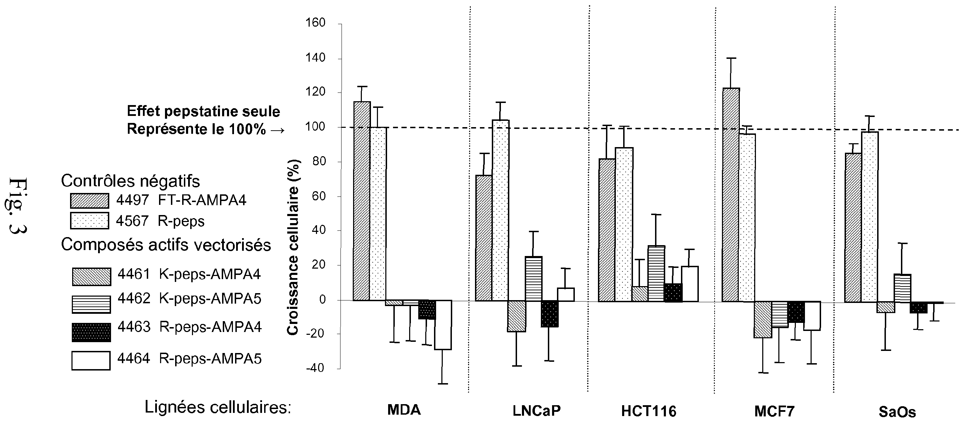

La Figure 3 décrit la croissance cellulaire (en pourcentage de nombre de cellules par rapport à un témoin pepstatine) de cellules cancéreuses (MDA-MB-231, MCF7, LNCaP, HCT116, SaOs2) en fonction de leur incubation pendant 5 jours avec 1 oligomère d'AMPA (4497), de la [Val¹]-Pepstatine reliée à de l'arginine (4567) et 4 conjugués [Val¹]-Pepstatine-oligomère d'AMPA reliés à un résidu lysine ou arginine(4461, 4562, 4463, 4564). Les pourcentages négatifs correspondent à une diminution du nombre de cellules par rapport à l'ensemencement cellulaire initial.

FT, représente la carboxyfluorescéine qui permet de contrôler la pénétration par mesure de fluorescence.

La présente invention est décrite plus en détail à l'aide des exemples suivants, qui sont donnés à titre d'illustration et auxquels l'invention n'est pas limitée.

### Exemple 1 : Oligomères synthétisés

Des oligomères de différentes tailles ont été synthétisés. Ces oligomères peuvent être de nature hydrophobe, hydrophile ou amphipatique en fonction des mimes utilisés. Ils ont la capacité de traverser les membranes cellulaires et sont probablement internalisés par voie endosomale. Afin de suivre l'internalisation des oligomères dans les cellules par microscopie à fluorescence, l'isothiocyanate de fluorescéine a été utilisé. La sonde fluorescente (fluorescéine) est introduite, soit directement sur l'extrémité N-terminale du poly-CM (CM= mime contraint) ancré à la résine (composés 1 à 3 et 7) soit sur la fonction amine d'un espaceur (composés 4-6 et 8-9). L'importance de la configuration a été évaluée par la préparation de dérivés de DBT (composés 1-6) et de LBT (composés 7-9).

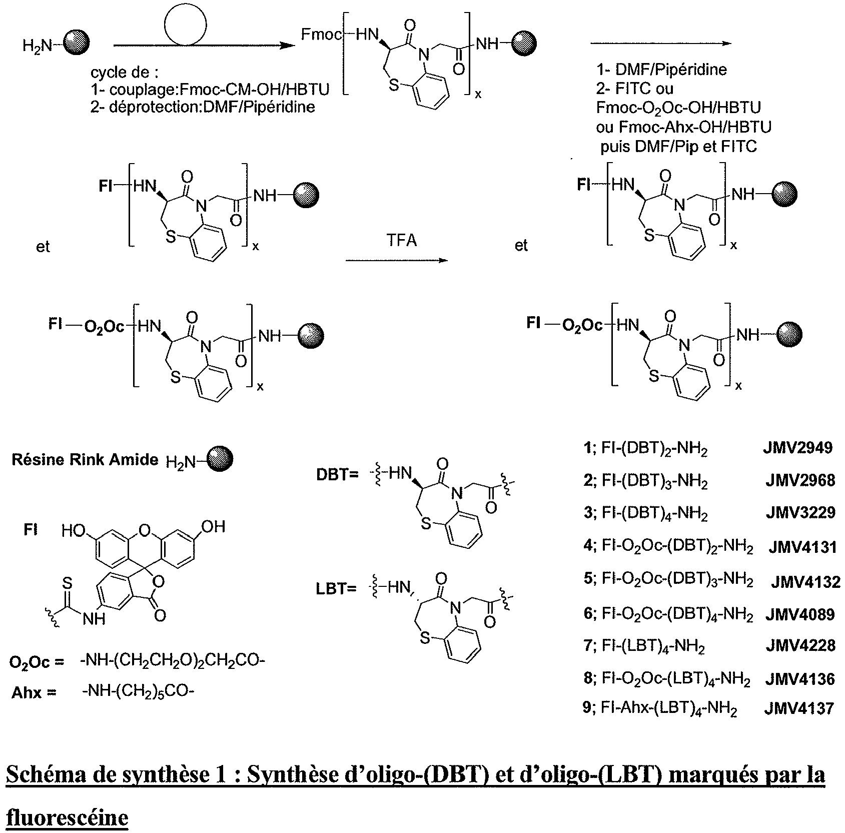

### Exemple 2 : Evaluation biologique

En première intention, les oligomères portant la sonde fluorescente sont testés pour leur capacité à pénétrer dans les cellules (lignées MDA-MB-231 de cancer du sein). Typiquement, deux expériences sont réalisées : mesure de la fluorescence totale par fluorimétrie après trypsinisation ménagée et lyse des cellules, et par microscopie confocale à fluorescence avec des marqueurs spécifiques des organites cellulaires.

Des tétra-oligomères de DBT sont capables de s'internaliser dans les cellules avec la même efficacité que l'octa-arginine (R8) décrite par le groupe de Wender (Standford, USA) et qui fait partie des CPP de référence décrits à ce jour dans la littérature. Des marqueurs spécifiques ont permis de démontrer que ces composés sont internalisés par voie endosomale et sont localisés à 16 heures dans des lysosomes. Cette localisation spécifique présente un intérêt certain pour cibler certaines pathologies : maladies lysosomales, maladie d'Alzheimer, certains cancers.

En premier lieu, la quantité absorbée et la fraction internalisée des oligomères ont été analysées (Figure 1A). Pour cette raison, des cellules de la lignée MDA-MB-231 de cancer du sein ont été incubées dans un milieu contenant 10⁻⁵ M de composés (1-6 du schéma 1) marqués par fluorescence, pendant 3 heures. L'octa-arginine marquée par de la fluorescéine a été utilisée en tant que contrôle positif, et de la carboxyfluorescéine en tant que contrôle négatif Après incubation des oligomères de DBT, les cellules ont été lavées avec un tampon phosphate. Pour déterminer les fractions internalisées des composés, un traitement de 5 minutes à la trypsine a été réalisé afin d'éliminer les composés restés liés aux membranes cellulaires lors de la transduction. L'émission de fluorescence a été mesurée sur un lecteur pour microplaques.
Tel qu'il est montré sur la figure 1A, les quantités d'oligomères internalisés sont importantes et correspondent au moins à 40 % de la totalité des oligomères retenus sur les cellules. L'intensité de fluorescence la plus importante ayant été observée est pour le composé DBT₄ (JMV3229), l'intensité de fluorescence des oligomères DBT₃ (JMV2968) et DBT₂ (JMV2949) étant largement inférieure. Ces derniers présentent des capacités d'internalisation au moins 8 fois inférieures à celle des dérivés de DBT₄. L'absorption cellulaire apparaît dépendante de la longueur de l'oligomère avec une émission de fluorescence croissant avec la taille de l'oligomère. En outre les dérivés de DBT₄ sont aussi efficaces que l'octa-arginine malgré le fait que les DBT₄ ne sont pas chargés. Une meilleure efficacité du DBT4 par rapport aux oligomères plus courts peut être associée à une augmentation de l'hydrophobicité et/ou une structuration progressive de l'oligomère. Les composés JMV4137 et JMV4228 construits par polymérisation du monomère L-benzothiazépinone (LBT) sont aussi efficaces que leurs contreparties D, indiquant que la configuration du fragment DBT n'est pas importante. Ce résultat devrait exclure l'implication d'un mécanisme récepteur-dépendant pour l'internalisation cellulaire de DBT₄. L'introduction d'un espaceur à l'extrémité N-terminale des oligomères (composés JMV4089, JMV4136 et JMV 4137) n'induit pas de perturbation importante du système.

### Exemple 3 : Analyse du facteur température

Une analyse de la dépendance de l'internalisation vis-à-vis de la température a été réalisée en incubant des cellules avec 10⁻⁵ M de JMV3229 à 4°C et 37°C (figure 1B). L'absorption cellulaire est diminuée par trois à basse température suggérant un passage endocytotique dépendant de l'énergie, plutôt qu'une pénétration passive de l'oligomère dans la cellule.

### Exemple 4 : Analyse dose-réponse

Une analyse dose-réponse a été réalisée pour comparer l'absorption des oligomères de DBT vis-à-vis de l'octa-arginine R₈ (Figure 1C). La pénétration de tous les composés apparaît être dose-dépendante.

### Exemple 5 : Etude cinétique

L'étude cinétique faite avec le DBT le plus efficace, le JMV 3229, (Figure 1D) montre que son entrée cellulaire est aussi rapide que celle de l'octa-arginine R₈, mais aucun plateau n'est observé à 3 heures. En contraste, l'internalisation de JMV3229 augmente jusqu'à 16 heures pour atteindre une concentration 11 fois supérieure à celle de l'octa-arginine R₈.

### Exemple 6 : Analyse de la cytotoxicité

La cytotoxicité de cette nouvelle classe de molécules a été également déterminée dans des cellules de lignée MDA-MB-231 en utilisant un test diagnostic de viabilité cellulaire, le MTT. Après une incubation de 5 jours, les composés aux concentrations utilisées pour les études d'internalisation, ne présentent aucun effet significatif sur la viabilité cellulaire, sauf pour le composé JMV4131 à 10⁻⁵M. Ces résultats indiquent que les poly-DBT ne présentent pas de risque d'effets cytotoxiques propres ce qui est favorable pour leur utilisation en tant que vecteurs pour la délivrance de médicament.

### Exemple 7 : analyse par CLSM

L'analyse par microscopie à scanner laser confocal (CLSM) a aussi été réalisée sur des cellules vivantes pour déterminer l'internalisation et la distribution intracellulaire. Cette expérience a été associée avec une étude cinétique de l'internalisation des oligomères de DBT.

La co-coloration avec un marqueur de membrane (marquage des radeaux lipidiques) a été réalisée pour vérifier que le composé JMV3229 hydrophobe n'a pas été emprisonné dans la membrane. Il a été observé par analyse CLSM et en accord avec les résultats de la Figure 1D que l'internalisation de ce composé dans les organites cellulaires augmentait jusqu'à 16 heures puis diminuait. De plus, les résultats montrent que le JMV3229 non-cationique a une internalisation comparable à celle de l'octa-arginine R₈ à 3 heures.

L'internalisation et la localisation des oligomères de LBT, JMV4137 et JMV4089 sont similaires à celles du composé JMV3229

Afin d'avoir un meilleur aperçu du mécanisme de translocation des oligomères DBT, des co-colorations ont été faites avec des marqueurs fluorescents divers pour des composants sub-cellulaires, tels que le noyau, la membrane plasmatique et les lysosomes. Les résultats montrent que JMV3229 est localisé dans des vésicules non-marquées (endosomales) à 3 heures et devient essentiellement co-localisé avec un marqueur lysosomal après 16 heures.

Des résultats similaires ont été obtenus avec les oligomères JMV4089 et JMV4137.

L'effet de la température sur l'internalisation du composé JMV3229 a été déterminé. A 4°C la majorité du composé JMV3229 est co-localisé avec le marqueur de membranes plutôt qu'internalisé. Ceci confirme les résultats présentés dans la figure 1B et suggère un mécanisme d'internalisation énergie-dépendante.

En conclusion, ces résultats indiquent le potentiel de ces oligomères dans le ciblage du compartiment endolysosomal. Bien que l'adressage lysosomal présente l'inconvénient d'une possible dégradation du principe actif par les enzymes lysosomales, des études récentes démontrent une utilité clinique intéressante à cibler une thérapie vers ce compartiment. En conclusion l'efficacité prouvée de la pénétration cellulaire des oligomères courts de D- ou L-BT, offre une nouvelle classe de vecteurs possédant la particularité d'être des transporteurs non-cationiques.

### Exemple 8 : Bio-conjugués vecteurs-drogues

Nous avons montré que les tétra-oligomères de DBT sont capables de rentrer dans les cellules avec la même efficacité que l'octa-arginine (R8) un des meilleurs CPP décrit dans la littérature. Ces composés sont internalisés par voie endosomale et sont localisés à 16 heures dans les lysosomes. Cette localisation spécifique présente un intérêt certain pour cibler certaines pathologies comme les maladies lysosomales, la maladie d'Alzheimer et certains cancers. Ces composés originaux et non toxiques ont la particularité d'être non cationiques, ce qui peut être un avantage pour la vectorisation de molécules actives ciblant le système nerveux central.

L'application de ces vecteurs dans le cadre d'une pathologie tumorale est actuellement explorée. Nous nous intéressons en particulier à certains cancers, notamment du sein, dans lesquels la cathepsine D, une protéase du système endo-lysosomal, est surexprimée et contribue à la prolifération des cellules tumorales. Nous étudions l'effet d'un inhibiteur naturel et puissant de cette enzyme, la pepstatine A, sur la prolifération de diverses cellules tumorales. L'inhibiteur seul, du fait d'une trop faible pénétration intra-cellulaire, est inactif. Nous avons montré que des bio-conjugués formés par des inhibiteurs de cathepsine D (plus particulièrement dans notre cas décrit, l'analogue [Val¹]-Pepstatine de la pepstatine ou des analogues moins actifs tels que le composé incorporant la gammaleucine à la place de la statine) associés à des oligomères de mimes contraints de dipeptides (plus particulièrement dans notre cas l'acide 2-aminométhyl-phényl-acétique ou AMPA) pouvait pénétrer dans les cellules, se localisant préférentiellement dans le système endo-lysosomal. De plus, ces conjugués sont capables d'inhiber totalement à 10⁻⁵ M la prolifération des lignées tumorales testées (cf. figures 2 et 3).

| **Numéro JMV** | **Formule Chimique** |
|---|---|
| **4463** | Ac-D-Arg-O₂Oc-([Val¹]-Pepstatine)-(AMPA)₄-NH₂ |
| 4461 | Ac-D-Lys-O₂Oc-([Val¹]-Pepstatine)-(AMPA)₄-NH₂ |
| 4462 | Ac-D-Arg-O₂Oc-([Val¹]-Pepstatine)-(AMPA)₅-NH₂ |
| 4464 | Ac-D-Arg-O₂Oc-([Val¹]-Pepstatine)-(AMPA)₅-NH₂ |
| 4567 | Ac-D-Arg-O₂Oc-([Val¹]-Pepstatine)-NH₂ |
| 4564 | Ac-D-Arg-O₂Oc-Val-Val-Val-γLeu-Ala-γLeu-(AMPA)₄-NH₂ |

| | |
|---|---|
| [Val¹]-Pepstatine = Val-Val-Val-Sta-Ala-Sta- (avec résidu statine = Sta = (*3S, 4S*)-4-amino-3-hydroxy-6-methyl-heptanoyle O₂Oc = 8-amino-3,6-dioxaoctanoyle Gammaleu = -Val-Val-Val-γLeu-Ala-γLeu- (avec γLeu = (*4S*)4-amino-6-methyl-heptanoyle) | |

La construction des composés est :
« Résidus hydrophiles (pour problème de solubilité, peuvent être Arg, Lys, O2Oc, ou autres) - Inhibiteurs - vecteurs » (exemple : Ac-D-Arg-O2Oc-[Val¹]-Pepstatine -(AMPA)₄-NH2.

## Revendications

1. Utilisation d'un oligomère de formule I' :
-X₁-Aₙ-X₂- (I')
dans laquelle :
- les unités récurrentes A, indépendamment identiques ou différentes les unes des autres, représentent des mimes contraints de dipeptides ou de tripeptides choisies parmi les formules (V) et (VI) suivantes : 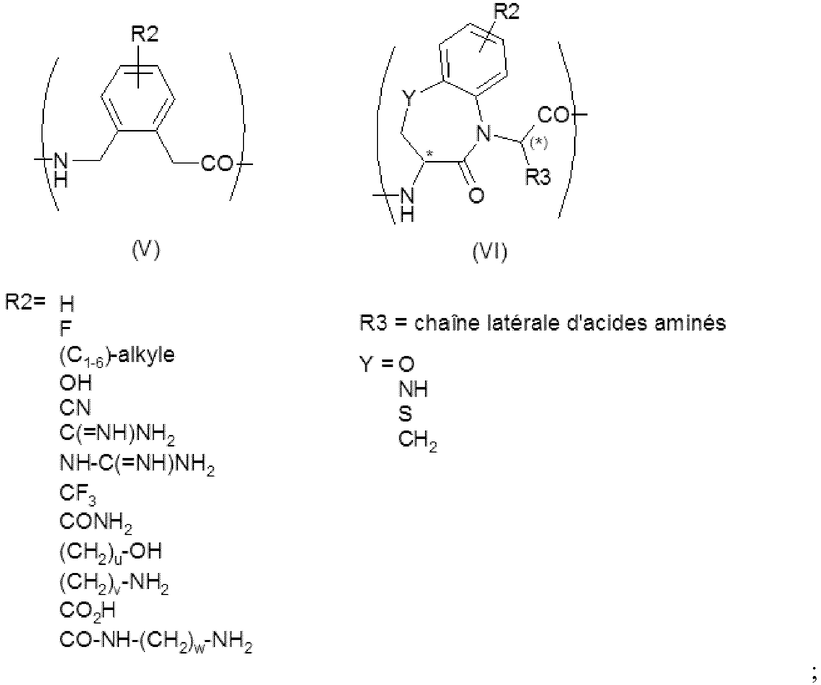
- n est un nombre entier compris entre 2 et 40 ;
- X₁ et X₂, indépendamment l'un de l'autre, représentent un groupe espaceur ou une liaison ;
- u, v et w étant des nombres entiers compris entre 0 et 10 ; en tant qu'agent de vectorisation.

2. Utilisation selon la revendication 1, **caractérisée en ce que** n est compris entre 4 et 8.

3. Utilisation selon l'une quelconque des revendications précédentes, **caractérisée en ce qu'**il s'agit d'un hétérooligomère où -Aₙ- présente la formule (VII) :
-[B-D]ₙ- (VII)
dans lequel :
- - les unités récurrentes B et D différentes l'une de l'autre, représentent des mimes contraints de dipeptides ou de tripeptides choisies parmi les formules (V) et (VI) ;

4. Utilisation selon la revendication 3, **caractérisée en ce que** les deux unités B et D sont des unités de formule (VI) différentes l'une de l'autre.

5. Utilisation selon la revendication 4, **caractérisée en ce que** l'un desdits dérivés de formule (VI) est un composé de formule (VIII) :

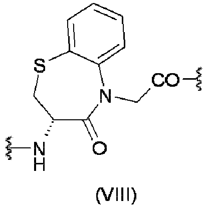

6. Utilisation selon l'une quelconque des revendications précédentes, **caractérisée en ce que** les groupes espaceurs X₁ et X₂, indépendamment l'un de l'autre sont une liaison ou choisis parmi les groupes suivants:
-NH-(CH₂CH₂O)₂CH₂CO- (IX)
-NH-(CH₂)₅CO- (X)

7. Oligomère représenté par la formule générale (I):
R₆-X₁-(A)ₙ-X₂-R₇ (I)
dans laquelle :
- l'un au moins de R₆ et R₇, est la pepstatine, la [Val¹]-pepstatine, ou un marqueur;
- dans le cas où R₆ est la pepstatine, la [Val¹]-pepstatine, ou un marqueur, R₇ est choisi parmi les groupes hydroxy, C₁-C₆ alcoxy, aryl-(C₁-C₆ alcoxy), ou NH₂ ou R₇ représente la pepstatine, la [Val¹]-pepstatine, ou un marqueur identique ou différent de R₆ ; dans le cas où R₇ est la pepstatine, la [Val¹]-pepstatine, ou un marqueur, R₆ est choisi parmi un atome d'hydrogène, un groupe C₁-C₆ alkyle, un groupe aryl-(C₁-C₆ alkyl)-, ou R₆ est la pepstatine, la [Val¹]-pepstatine, ou un marqueur identique ou différent de R₇ ;
- les unités récurrentes -A-, indépendamment identiques ou différentes les unes des autres, représentent des mimes contraints de dipeptides ou tripeptides choisies parmi les formules (V) et (VI) suivantes : 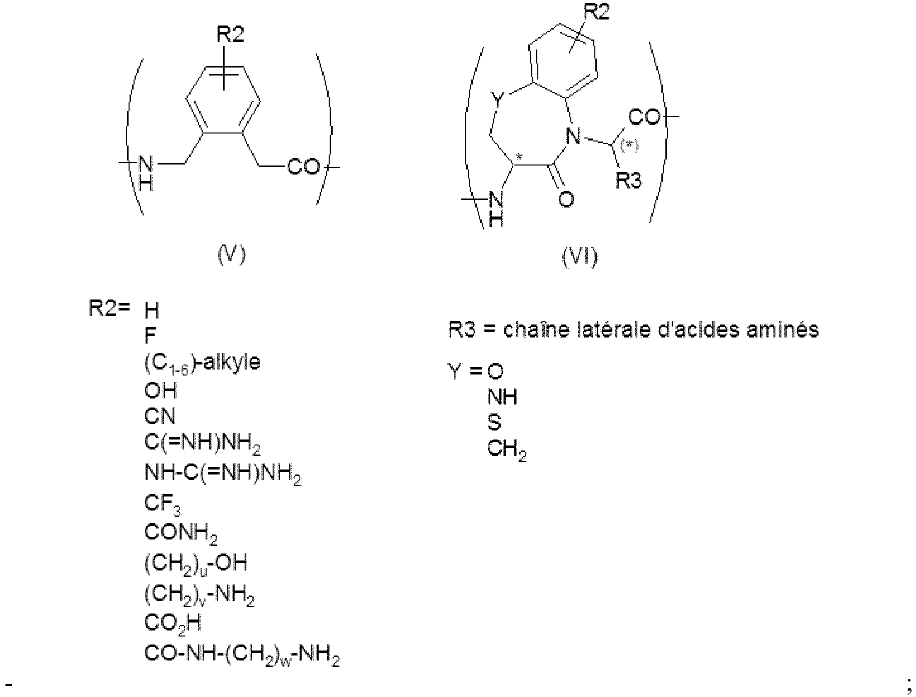
- n est un nombre entier compris entre 2 et 40 ;
- X₁ et X₂, indépendamment l'un de l'autre, représentent un groupe espaceur ou une liaison
- u, v et w étant des nombres entiers compris entre 0 et 10 ;
le marqueur est choisi parmi les marqueurs suivants :
fluorescéine, sel sodique de fluorescéine, 4',5'-Bis[*N*,*N-*bis(carboxyméthyl)-aminométhyl]fluorescéine, acide 6-[fluorescéine-5(6)-carboxamido]héxanoique, acide 6-[fluorescéine-5(6)-carboxamido]héxanoique ester *N*-hydroxysuccinimide de fluorescéin-5(6)-isothiocyanate, fluorescéin-α-D-N-acetylneuraminide-polyacrylamide, amidite de fluorescéine, fluorescéine-di(β-D-galactopyranoside), fluorescéin-di-(β-D-glucopyranoside), diacétate de fluorescéine, diacétate fluorescéin-5(6)-isothiocyanate, diacétate de fluorescéin-5-maleimide, diacétate de fluorescéin-6-isothiocyanate, dibutyrate de fluorescéine, fluorescéine dilaurate, sel de diphosphate de fluorescéine triammonium, acide fluorescéin-hyaluronic, isothiocyanate de fluorescéine- Dextran 500000-Conjugué, isomer I de la isothiocyanate de fluorescéine, isothiocyanate de fluorescéine-dextran, acétate de mercure-fluorescéine, chlorhydrate de mono-*p*-guanidinobenzoate-fluorescéine, *O*,*O'*-diacrylate de fluorescéine, fluorescéine *O*,*O*'-dimethacrylate, fluorescéine o-acrylate, fluorescéine *O*-méthacrylate, ester N-hydroxysuccinimide de fluorescéine, fluorescéine-5-thiosemicarbazide, fluorescéin-α-D-galactosamine polyacrylamide, fluorescéin-α-D-mannopyranoside-polyacrylamide, 4(5)-(iodoacétamido)-fluorescéine, 5-(Bromométhyl)fluorescéine, 5-(Iodoacétamido)fluorescéine, diacétate de l'ester *N-*succinimidyl-5-Carboxy-fluorescéine, diacétate de l'ester *N*-succinimidyl-6-Carboxy-fluorescéine, Aminophényl-fluorescéine, Biotin-4- fluorescéine, hydroxyphényl-fluorescéine, MTS-4- fluorescéine, chlorhydrate de poly(fluorescéine-isothiocyanate allylamine), poly(fluorescéine-*O*-acrylate), poly(fluorescéine-*O*-méthacrylate), acétate de PPHT-fluorescéine, chlorhydrate de 5-([4,6-dichlorotriazin-2-yl]amino) fluorescéine, chlorhydrate de 6-([4,6-dichlorotriazin-2-yl]amino) fluorescéine, poly[(méthylméthacrylate)-*co*-(fluorescéine-*O*-methacrylate)], poly[méthylméthacrylate-*co*-(fluorescéine *O*-acrylate)], 5(6)-(Biotinamidohexanoylamido)pentylthioureidylfluorescéine, N-(5-fluorescéinyl)maleimide, sel disodique de Mercure-dibromo-fluorescéine, fluorescéin-di-[méthylène-N-méthylglycine], sel disodique de 2',4',5',7'-tétrakis-(acétoxymercuro)-fluoroscéine, l'érythrosin B, l'éthyl eosin, 5-carboxy fluorescéine, ester *N*-succinimidyl de 5-carboxy fluorescéine ester perchlorate de, rhodamine B octadecyl, ester N-hydroxysuccinimide de 6-Carboxy-fluorescéine, dibenzyl fluorescéine, rhodol, 6-amino fluorescéine, rhodamine 6G, la rhodamine B et la rhodamine 123.

8. Oligomère selon la revendication 7, **caractérisé en ce que** n est compris entre 4 et 8.

9. Oligomère selon l'une quelconque des revendications 7 ou 8, **caractérisé en ce que** les groupes espaceurs X₁ et X₂ indépendamment l'un de l'autre sont une liaison ou choisis parmi les groupes suivants :
-NH-(CH₂CH₂O)₂CH₂CO- (IX)
-NH-(CH₂)₅CO- (X)

10. Oligomère selon l'une quelconque des revendications 7 à 9 en tant que médicament.

11. Oligomère selon l'une quelconque des revendications 8 à 10 pour son utilisation dans le traitement d'un cancer dans lequel la cathepsine D est surexprimée.

12. Procédé de préparation d'un oligomère selon l'une quelconque des revendications 7 à 9, **caractérisé par** les étapes successives suivantes :
a) polymérisation par une stratégie de synthèse peptidique sur support solide, comprenant la réaction de l'unité de mime contraint de dipeptides ou tripeptides de formule (XI') suivante :
P-A-OH (XI')
dans lesquels le groupement P est un groupement N-protecteur, l'unité récurrente - A étant telle que définie dans la revendication 7, pour synthétiser le produit (XII) suivant :
H-(A)ₙ-X₂-R₈-(SS) (XII)
dans lequel,
- n et X₂ sont tels que définis dans la revendication 7 ;
- R₈ est un groupe précurseur de R₇ avant clivage, R₇ est tel que défini dans la revendication 7 ;
- (SS) est le support solide ;
a2) éventuellement, si X₁ n'est pas une liaison, réaction de couplage entre P-X₁-OH, dans lequel le groupement P est un groupement N-protecteur et X₁ est tel que défini dans la revendication 7, et l'amine N-terminale du produit (XII), suivie d'une étape de déprotection de l'extrémité N-terminale pour synthétiser le produit (XII') suivant :
H-X₁-(A)ₙ-X₂-R₈-(SS) (XII')
b) réaction de couplage entre la pepstatine ou la [Val¹]-pepstatine et l'amine N-terminale du produit (XII'), pour synthétiser le produit (XIII) suivant :
(PA)-X₁-(A)ₙ-X₂-R₈-(SS) (XIII)
dans lequel,
(PA) est la pepstatine ou la [Val¹]-pepstatine,
c) une réaction de clivage permettant de libérer l'oligomère de formule (I) du support solide à partir du produit de formule (XIII).

13. Procédé selon la revendication 12, **caractérisé par** une étape (a1) antérieure à l'étape (a) de couplage d'un groupe espaceur X₂ sur le support solide.

## Patentansprüche

1. Verwendung eines Oligomers der Formel I' :
-X₁-Aₙ-X₂- (I')
wobei:
- die Wiederholungseinheiten A, unabhängig voneinander, gleich oder unterschiedlich sind, und eingeschränkte Mimen von Dipeptiden oder Tripeptiden darstellen, ausgewählt aus den folgenden Formeln (V) und (VI): 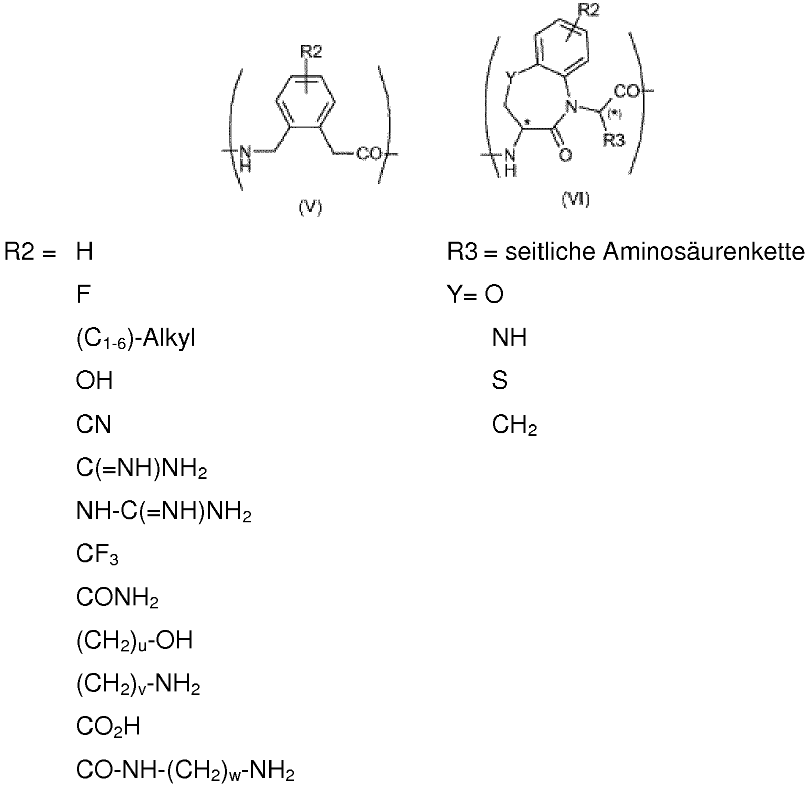
- n eine ganze Zahl zwischen 2 und 40 ist;
- X₁ und X₂, unabhängig voneinander, eine Spacergruppe oder eine Bindung darstellen;
- u, v, und w ganze Zahlen zwischen 0 und 10 sind;
als Vektorisierungsagens.

2. Verwendung nach Anspruch 1, **dadurch gekennzeichnet, dass** n zwischen 4 und 8 liegt.

3. Verwendung nach einem der vorangehenden Ansprüche, **dadurch gekennzeichnet, dass** es sich um ein Heterooligomer handelt, wobei -Aₙ- die Formel (VII) aufweist:
-[B-D]ₙ- (VII)
wobei:
die Widerholungseinheiten B und D, unterschiedlich voneinander, eingeschränkte Mimen von Dipeptiden oder Tripeptiden darstellen, ausgewählt aus der Formeln (V) und (VI).

4. Verwendung nach Anspruch 3, **dadurch gekennzeichnet, dass** die zwei Einheiten B und D Einheiten der Formel (VI) sind, die unterschiedlich voneinander sind.

5. Verwendung nach Anspruch 4, **dadurch gekennzeichnet, dass** ein von den Derivaten der Formel (VI) eine Verbindung der Formel (VIII) ist:

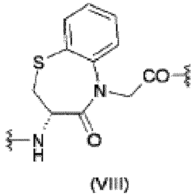

6. Verwendung nach einem der vorangehenden Ansprüche, **dadurch gekennzeichnet, dass** die Spacergruppen X₁ und X₂, unabhängig voneinander, eine Bindung oder ausgewählt aus den folgenden Gruppen sind:
-NH-(CH₂CH₂O)₂CH₂CO- (IX)
-NH-(CH₂)₅CO- (X)

7. Oligomer dargestellt durch die allgemeine Formel (I):
R₆-X₁-(A)ₙ-X₂-R₇ (I)
wobei:
- ein von wenigstens R₆ und R₇ Pepstatin, [Val¹]-Pepstatin oder ein Marker ist;
- im Fall R₆ Pepstatin, [Val¹]-Pepstatin oder ein Marker ist, R₇ ausgewählt ist aus den Gruppen Hydroxy, C₁-C₆-Alkoxy, Aryl-(C₁-C₆-alkoxy), oder NH₂, oder R₇ Pepstatin, [Val¹]-Pepstatin oder einen von R₆ identischen oder unterschiedlichen Marker darstellt; im Fall R₇ Pepstatin, [Val¹]-Pepstatin oder ein Marker ist, R₆ ausgewählt ist aus den Gruppen Hydroxy, C₁-C₆-Alkoxy, Aryl-(C₁-C₆-Alkoxy), oder NH₂, oder R₆ Pepstatin, [Val¹]-Pepstatin oder einen von R₇ identischen oder unterschiedlichen Marker darstellt;
- die Wiederholungseinheiten A, unabhängig voneinander, gleich oder unterschiedlich sind, und eingeschränkte Mimen von Dipeptiden oder Tripeptiden darstellen, ausgewählt aus den folgenden Formeln (V) und (VI): 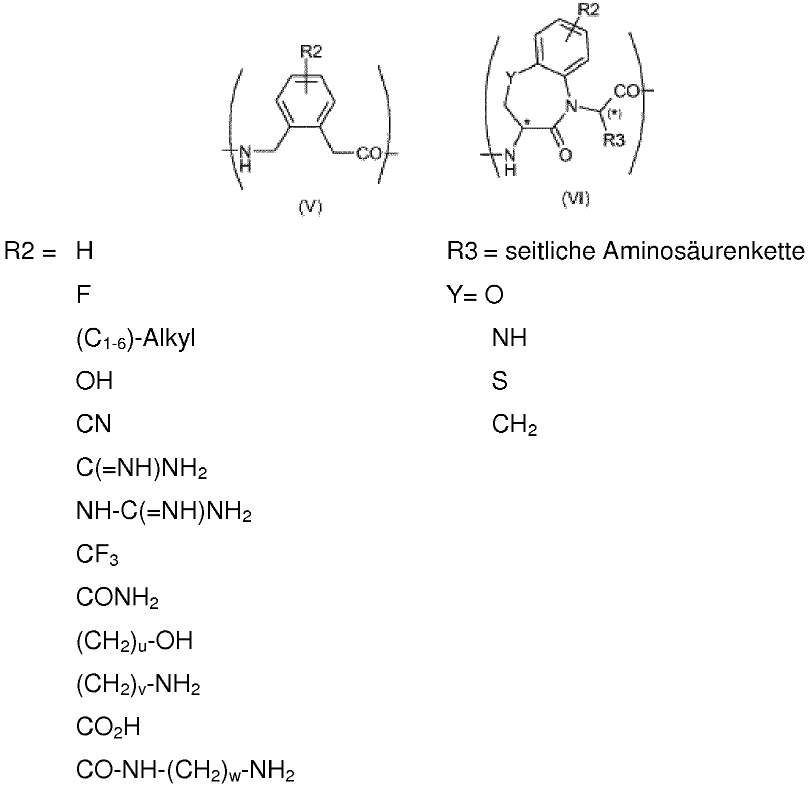
- n eine ganze Zahl zwischen 2 und 40 ist;
- X₁ und X₂, unabhängig voneinander, eine Spacergruppe oder eine Bindung darstellen;
- u, v, und w ganze Zahlen zwischen 0 und 10 sind;
- der Marker ausgewählt ist aus den folgenden Marker:
Fluorescein, Fluorescein Natriumsalz, 4', 5'-Bis[*N*,*N*-bis(carboxymethyl)-aminomethyl]fluorescein, 6-[Fluorescein-5(6)-carboxamido]hexansäure, 6-[Fluorescein-5-(6)carboxamido]hexansäure, Fluorescein-5(6)isothiocyanat *N-*hydroxysuccinimidester, Fluorescein-α-D-N-acetylneuraminid-polyacrylamid, Fluoresceinamidit, Fluorescein-di(β-D-galactopyranosid), Fluorescein-di-(β-D-glucopyranosid), Fluoresceindiacetat, Fluorescein-5(6)-isothiocyanatdiacetat, Fluorescein-5-maleimiddiacetat, Fluorescein-6-isothiocyanatdiacetat, Fluoresceindibutyrat, Fluoresceindilaurat, Fluoresceindiphosphat Triammoniumsalz, Fluoresceinhyaluronsäure, Fluoresceinisothiocyanatdextran-Konjugat-500000, Isomer I von Fluoresceinisothiocyanat, Fluoresceindextranisothiocyanat, Quecksilber-Fluoresceinacetat, Mono-p-guanidinobenzoat-fluoresceinhydrochlorid, *O*,*O*'-Fluoresceindiacrylat, *O*,*O*'-Fluoresceindimethacrylat, *O*-Fluoresceinacrylat, *O*- Fluoresceinmethacrylat, Fluorescein-*N*-hydroxysuccinimidester, Fluorescein-5-thiosemicarbazid, Fluorescein-α-D-galactosaminpolyacrylamid, Fluorescein-α-D-mannopyranosipolyacrylamidgel, 4(5)-(lodacetamido)fluorescein, 5-(Brommethyl)fluorescein, 5- (lodacetamido)fluorescein, *N*-Succinimidyl-5-carboxyfluoresceindiacetatester, *N*-Succinimidyl-6-carboxyfluoresceindiacetatester, Aminophenylfluorescein, Biotin- 4-fluorescein, Hydroxyphenylfluorescein, MTS-4-fluorescein, Poly-(fluoresceinisothiocyanatallylamin)hydrochlorid, Poly(fluorescein-*O*-acrylat), Poly (fluorescein-*O*-methacrylat), PPHT-fluoresceinacetat, 5-([4,6-Dichlortriazin-2-yl] amino)fluoresceinhydrochlorid, 6-([4,6-Dichlortriazin-2-yl]amino)fluoresceinhydrochlorid, Poly[(methylmethacrylat)-*co*-(fluorescein-*O-*methacrylat)], Poly[methylmethacrylat-*co*-(fluorescein-*O*-acrylat)], 5(6)-(Biotinamidohexanoylamido)pentylthioureidylfluorescein, N-(5-Fluoresceinyl) maleimid, Quecksilber-dibromfluorescein Dinatriumsalz, Fluorescein-di-[methylen-N-methylglycin], 2',4',5',7'-tetrakis-(acetoxymercuro)-fluoroscein, Erythrosin B, Ethyleosin, 5-Carboxyfluorescein, 5-Carboxyfluoresceinperchloratester *N*-Succinimidylester, Rhodamin B Octadecyl, 6-Carboxyfluorescein N-hydroxysuccinimidester, Dibenzylfluorescein, Rhodol, 6-Amino-fluorescein, Rhodamin 6G, Rhodamin B und Rhodamin 123.

8. Oligomer nach Anspruch 7, **dadurch gekennzeichnet, dass** n zwischen 4 und 8 liegt.

9. Oligomer nach einem der Ansprüche 7 oder 8, **dadurch gekennzeichnet, dass** die Spacergruppen X₁ und X₂, unabhängig voneinander, eine Bindung oder ausgewählt aus den folgenden Gruppen sind:
-NH-(CH₂CH₂O)₂CH₂CO- (IX)
-NH-(CH₂)₅CO- (X)

10. Oligomer nach einem der Ansprüche 7 bis 9 als Medikament.

11. Oligomer nach einem der Ansprüche 8 bis 10 zur Verwendung in der Behandlung von einem Krebs, in welchem Cathepsin D überexprimiert ist.

12. Verfahren zum Herstellen eines Oligomers nach einem der Ansprüche 7 bis 9, **gekennzeichnet durch** die nachstehenden aufeinanderfolgenden Schritte:
a) Polymerisierung mittels einer Peptidsynthesestrategie auf einem festen Träger, umfassend die Umsetzung der Einheit von eingeschränktem Mime von Dipeptiden oder Tripeptiden der folgenden Formel (XI'):
P-A-OH (XI')
wobei die Gruppe P eine N-Schutzgruppe ist, die Wiederholungseinheit A wie in Anspruch 7 definiert ist, um die folgende Verbindung (XII) zu synthetisieren:
H-(A)ₙ-X₂-R₈-(SS) (XII)
wobei
- n und X₂ wie in Anspruch 7 definiert sind;
- R₈ eine Vorläufergruppe von R₇ vor Spaltung ist, wobei R₇ wie in Anspruch 7 definiert ist;
- (SS) der feste Träger ist;
a2) optional, wenn X₁ keine Bindung ist, Kupplungsreaktion zwischen P-X₁-OH, worin die Gruppe P eine N-Schutzgruppe ist und X₁ wie in Anspruch 7 definiert ist, und der N-Terminus-Aminogruppe der Verbindung (XII), gefolgt von einem Entschützungsschritt des N-Terminus-Endes, um die folgende Verbindung (XII') zu synthetisieren:
H-X₁-(A)ₙ-X₂-R₈-(SS) (XII')
b) Kupplungsreaktion zwischen der Pepstatin oder [Val¹]-Pepstatin und der N-Terminus-Aminogruppe der Verbindung (XII'), um die folgende Verbindung (XIII) zu synthetisieren:
(PA)-X₁-(A)ₙ-X₂-R₈-(SS) (XIII)
wobei
(PA) Pepstatin oder [Val¹]-Pepstatin ist,
c) Spaltungsreaktion, die das Befreien des Oligomers der Formel (I) von dem festen Träger aus der Verbindung der Formel (XIII) ermöglicht.

13. Verfahren nach Anspruch 12, **gekennzeichnet durch** einen Schritt (a1) der Kupplung einer Spacergruppe X₂ auf dem festen Träger, wobei der Schritt (a1) vor dem Schritt (a) erfolgt.

## Claims

1. Use of an oligomer of formula I'
-X1-An-X2- (I')
where:
- the recurrent units A, each independently the same or different, are restricted mimetics of dipeptides or tripeptides selected from among the following formulas (V) and (VI): 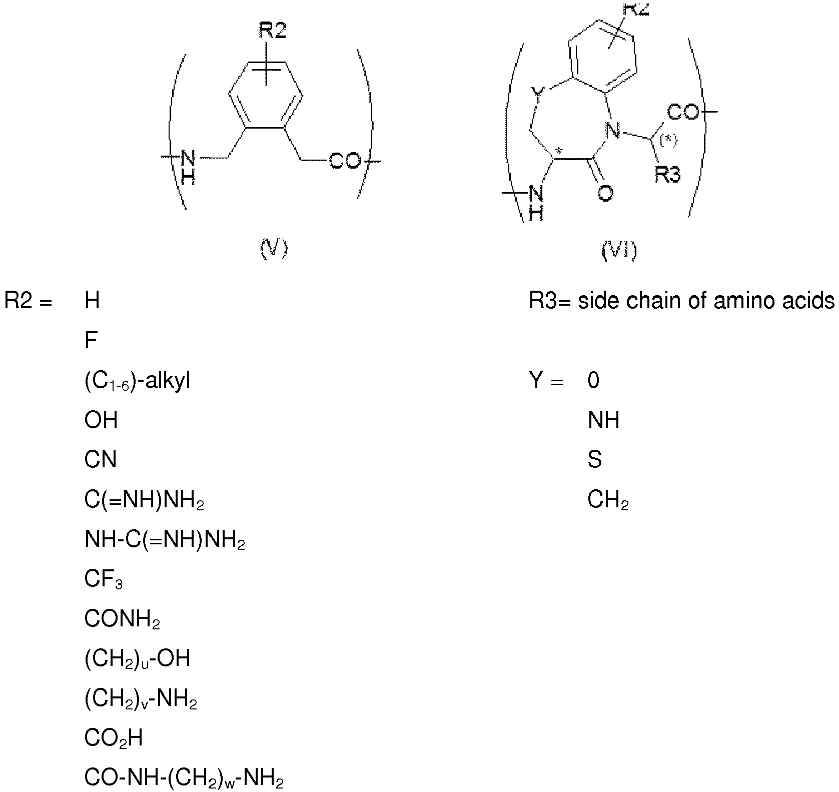
- n is an integer of between 2 and 40;
- X₁ and X₂ are each independently a spacer group or a linker;
- u, v and w being integers of between 0 and 10;
as vectorisation agent.

2. The use according to claim 1, **characterized in that** n is between 4 and 8.

3. The use according to any of the preceding claims, **characterized in that** it is a heterooligomer where -Aₙ- has the formula (VII):
-[B-D]ₙ- (VII)
where:
- the recurrent units B and D differing from one another represent restricted mimetics of dipeptides or tripeptides selected from among formulas (V) and (VI);

4. The use according to claim 3, **characterized in that** the two units B and D are units of formula (VI) that differ from one another.

5. The use according to claim 4, **characterized in that** one of said derivatives of formula (VI) is a compound of formula (VIII):

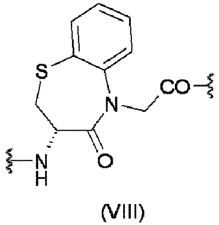

6. The use according to any of the preceding claims, **characterized in that** the spacer groups X₁ and X₂ each independently are a linker or selected from among the following groups:
-NH-(CH₂CH₂O)₂CH₂CO- (IX)
-NH-(CH₂)₅CO- (X)

7. Oligomer represented by the general formula (I):
R₆-X₁-(A)ₙ-X₂-R₇ (I)
where:
- at least one of R₆ and R₇ is pepstatin, [Val¹]-pepstatin, or a marker;
- if R₆ is pepstatin, [Val¹]-pepstatin or a marker, R₇ is selected from among hydroxy, C1-C₆ alkoxy, aryl-(C₁-C₆ alkoxy) groups, or NH₂, or R₇ is pepstatin, [Val¹]-pepstatin or a marker the same as or differing from R₆; if R₇ is pepstatin, [Val¹]-pepstatin or a marker, R₆ is selected from among a hydrogen atom, an alkyl C₁-C₆ group, an aryl-(C₁-C₆ alkyl)- group, or R₆ is pepstatin, [Val¹]-pepstatin or a marker the same as or differing from R₇;
- the recurrent units -A- each independently the same or different, are restricted mimetics of dipeptides or tripeptides selected from among the following formulas (V) and (VI): 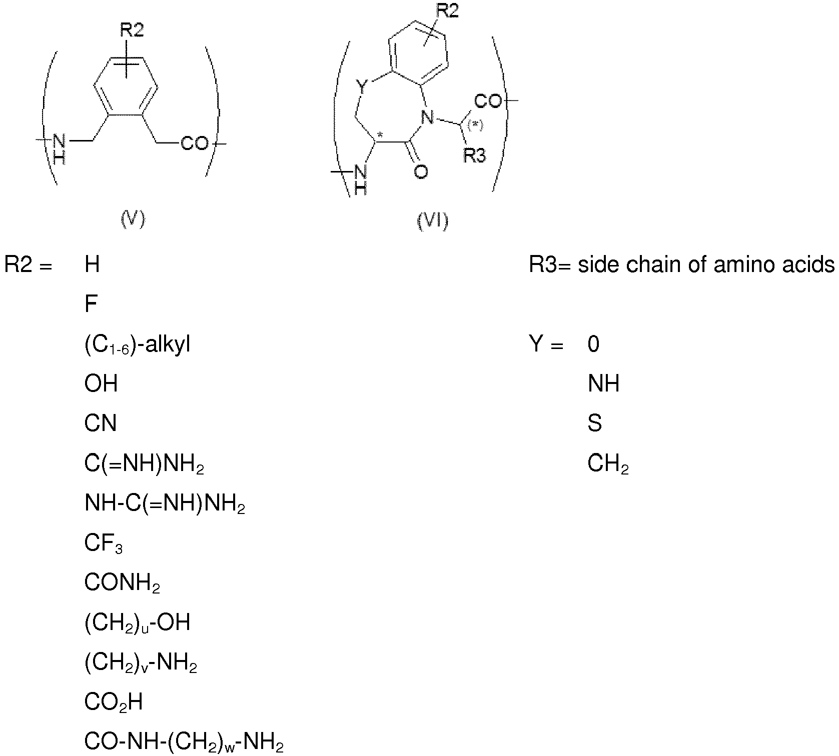
- n is an integer of between 2 and 40;
- X₁ and X₂ are each independently a spacer group or a linker;
- u, v and w being integers of between 0 and 10;
the marker is selected from among the following markers:
fluorescein, sodium salt of fluorescein, 4',5'Bis[N,*N-*bis(carboxymethyl)-aminomethyl]fluorescein, 6-[fluorescein-5(6)-carboxyamido]hexanoic acid, 6-[fluorescein-5(6)-carboxamido] acid, *N*-hydroxysuccinimide ester of fluorescein-5(6)-isothiocyanate, fluorescein-α-D-N-acetylneuraminide-polyacrylamide, fluorescein amidite, fluorescein-di(β-D-galactopyranoside), fluorescein-di-(β-D-glucopyranoside), fluorescein diacetate, fluorescein diacetate 5(6)-isothiocyanate, fluorescein diacetate 5-maleimide, fluorescein diacetate 6-isothiocyanate, fluorescein dibutyrate, fluorescein dilaurate, fluorescein diphosphate triammonium salt, fluorescein-hyaluronic acid, fluorescein isothiocyanate -Dextran 500000-Conjugate, fluorescein isothiocyanate I isomer, fluorescein-dextran isothiocyanate, fluorescein mercuric acetate, fluorescein mono-*p*-guanidinobenzoate hydrochloride, fluorescein *O*,*O*'-diacrylate, fluorescein *O*,*O*'-dimethacrylate, fluorescein *O*-acrylate, fluorescein *O*-methacrylate, fluorescein N-hydroxysuccinimide ester, fluorescein-5-thiosemicarbazide, fluorescein-α-D-galactosamine polyacrylamide, fluorescein-α-D-mannopyranoside-polyacrylamide, 4(5)-(iodoacetamido)-fluorescein, 5-(Bromomethyl)fluorescein, 5-(lodoacetamido)fluorescein, 5-Carboxy-fluorescein diacetate N-succinimidyl ester, 6-Carboxy-fluorescein diacetate N-succinimidyl ester, Aminophenyl-fluorescein, Biotin-4-fluorescein, hydroxyphenyl-fluorescein, MTS-4-fluorescein, poly(fluorescein-isothiocyanate allylamine) hydrochloride, poly(fluorescein-*O*-acrylate), poly(fluorescein-*O*-methacrylate), PPHT-fluorescein acetate, 5-([4,6-dichlorotriazin-2-yl]amino) fluorescein hydrochloride, 6-([4,6-di chlorotriazin-2-yl]amino) fluorescein hydrochloride, poly[(methylmethacrylate)-co-(fluorescein-*O*-methacrylate)], poly[methylmethacrylate-*co*-(fluorescein *O*-acrylate)], 5(6)-(Biotinamidohexanoylamido)pentylthioureidylfluorescein, N-(5-fluoresceinyl)maleimide, Mercury-dibromo-fluorescein disodium salt, fluorescein-di-[methylene-N-methylglycine], 2',4',5',7'-tetrakis-(acetoxymercuro)-fluoroscein disodium salt, erythrosine B, ethyl eosin, 5-carboxy fluorescein, 5-carboxy fluorescein *N*-succinimidyl ester, rhodamine B octadecyl ester perchlorate, 6-carboxyfluorescein N-hydroxysuccinimide ester, dibenzylfluorescein, rhodol, 6-amino fluorescein, rhodamine 6G, rhodamine B or rhodamine 123.

8. The oligomer according to claim 7, **characterized in that** n is between 4 and 8.

9. The oligomer according to any of claims 7 or 8, **characterized in that** the spacer groups X₁ and X₂ are each independently a linker or selected from among the following groups:
NH-(CH₂CH₂O)₂CH₂CO- (IX)
-NH-(CH₂)₅CO- (X)

10. The oligomer according to any of claims 7 to 9 as medicinal product.

11. The oligomer according to any of claims 8 to 10 for use thereof in the treatment of a cancer in which cathepsin D is overexpressed.

12. Method for preparing an oligomer according to any of claims 7 to 9, **characterized by** the following successive steps:
a) polymerization via peptide synthesis strategy on a solid substrate, comprising the reaction of the restricted mimetic unit of dipeptides or tripeptides of following formula (XI'):
P-A-OH (XI')
where the P group is an N-protective group, the recurrent unit -A being such as defined in claim 7, to synthesize the following product (XII):
H-(A)ₙ-X₂-R₈-(SS) (XII)
where:
- n and X₂ are such as defined in claim 7;
- R₈ is a precursor group of R₇ before cleavage, R₇ is such as defined in claim 7;
- (SS) is the solid substrate;
a2) optionally, if X₁ is not a linker, coupling reaction between P-X₁-OH, where the P group is an N-protective group and X₁ is such as defined in claim 7, and the N-terminal amine of product (XII), followed by a deprotection step of the N-terminal end to synthesize the following product (XII'):
H-X₁-(A)ₙ-X₂-R₈-(SS) (XII')
b) coupling reaction between pepstatin or [Val¹]-pepstatin and the N-terminal amine of product (XII') to synthesize the following product (XIII):
(PA)-X₁-(A)ₙ-X₂-R₈-(SS) (XIII)
where:
(PA) is pepstatin or [Val¹]-pepstatin;
c) cleavage reaction allowing release of the oligomer of formula (I) from the solid substrate, with the product of formula (XIII).

13. The method according to claim 12, **characterized by** a step (a1) prior to step (a) for the coupling of a spacer group X₂ on the solid substrate.
